# EUROPÄISCHE PATENTSCHRIFT

(11) **EP 1 183 378 B1**
(45) Veröffentlichungstag und Bekanntmachung des Hinweises auf die Patenterteilung: **26.08.2015**
(21) Anmeldenummer: 00943614.8
(22) Anmeldetag: 19.05.2000
(51) Int. Cl.: C12N 15/82, C12N 15/29, A01H 5/00, A01N 65/00

(54) **NUKLEOTIDSEQUENZ ZUR ERHÖHUNG DER ABWEHRREAKTION EINER PFLANZE GEGEN PATHOGENBEFALL**
NUCLEOTIDE SEQUENCE FOR INCREASING THE DEFENSE REACTION OF A PLANT AGAINST INFECTION BY A PATHOGEN
SEQUENCE NUCLEOTIDIQUE POUR AUGMENTER LA REACTION DE DEFENSE D'UNE PLANTE CONTRE UNE AFFECTION PAR DES PATHOGENES

(30) Priorität: 21.05.1999 DE 19923571
(43) Veröffentlichungstag der Anmeldung: 06.03.2002
(73) Patentinhaber: KWS Saat AG, 37574 Einbeck (DE)
(72) Erfinder: STAHL, Dietmar, J., 37574 Einbeck (DE)
(74) Vertreter: Westhoff, Markus
(86) Internationale Anmeldenummer: PCT/DE2000/001589
(87) Internationale Veröffentlichungsnummer: WO 2000/071732

(56) Entgegenhaltungen:
- WO-A-00/29592
- LOAKE G J ET AL: "COMBINATION OF H-BOX CCTACCN-7CT AND G-BOX CACGTG CIS ELEMENTS IS NECESSARY FOR FEED-FORWARD STIMULATION OF A CHALCONE SYNTHASE PROMOTER BY THE PHENYLPROPANOID-PATHWAY INTERMEDIATE P COUMARIC ACID" PROCEEDINGS OF THE NATIONAL ACADEMY OF SCIENCES OF THE UNITED STATES, Bd. 89, Nr. 19, 1992, Seiten 9230-9234, XP002159400 1992 ISSN: 0027-8424
- STRITTMATTER GUENTER ET AL: "Infections with various types of organisms stimulate transcription from a short promoter fragment of the potato gst1 gene." MOLECULAR PLANT-MICROBE INTERACTIONS, Bd. 9, Nr. 1, 1996, Seiten 68-73, XP000980090 ISSN: 0894-0282
- MARTINI N ET AL: "PROMOTER SEQUENCES OF A POTATO PATHOGENESIS-RELATED GENE MEDIATE TRANSCRIPTIONAL ACTIVATION SELECTIVELY UPON FUNGAL INFECTION" MOLECULAR AND GENERAL GENETICS,DE,SPRINGER VERLAG, BERLIN, Bd. 236, Nr. 2/03, 1993, Seiten 179-186, XP002044934 ISSN: 0026-8925
- RUSHTON, P.J. AND SOMSSICH, I.E.: "transciptional control of plant genes responsive to pathogens" CURRENT OPINION IN PLANT BIOLOGY, Bd. 1, Nr. 4, August 1998 (1998-08), Seiten 311-315, XP000980033 in der Anmeldung erwähnt
- SHAH JYOTI ET AL: "Identification of a salicylic acid-responsive element in the promoter of the tobacco pathogenesis-related beta-1,3-glucanase gene, PR-2d." PLANT JOURNAL, Bd. 10, Nr. 6, 1996, Seiten 1089-1101, XP002159401 ISSN: 0960-7412 in der Anmeldung erwähnt
- LIVNE BOAZ ET AL: "TMV-induced expression of tobacco beta-glucanase promoter activity is mediated by a single, inverted, GCC motif." PLANT SCIENCE (SHANNON), Bd. 130, Nr. 2, Dezember 1997 (1997-12), Seiten 159-169, XP000980511 ISSN: 0168-9452
- LOGEMANN ELKE ET AL: "Modes of expression and common structural features of the complete phenylalanine ammonia-lyase gene family in parsley." PROCEEDINGS OF THE NATIONAL ACADEMY OF SCIENCES OF THE UNITED STATES, Bd. 92, Nr. 13, 1995, Seiten 5905-5909, XP002159403 1995 ISSN: 0027-8424
- OHL S ET AL: "FUNCTIONAL PROPERTIES OF A PHENYLALANINE AMMONIA-LYASE PROMOTER FROM ARABIDOPSIS" PLANT CELL, Bd. 2, Nr. 9, 1990, Seiten 837-848, XP002159404 ISSN: 1040-4651 in der Anmeldung erwähnt
- RAVENTOS D ET AL: "A 20 bp cis-acting element is both necessary and sufficient to mediate elicitor response of a maize PRms gene" PLANT JOURNAL,GB,BLACKWELL SCIENTIFIC PUBLICATIONS, OXFORD, Bd. 7, Nr. 1, Januar 1995 (1995-01), Seiten 147-155, XP002107426 ISSN: 0960-7412 in der Anmeldung erwähnt
- GOTTSCHALK TINE E ET AL: "Immunolocalization and characterization of a beta-1,3-glucanase from sugar beet, deduction of its primary structure and nucleotide sequence by cDNA and genomic cloning." PLANT SCIENCE (SHANNON), Bd. 132, Nr. 2, 16. März 1998 (1998-03-16), Seiten 153-167, XP000980504 ISSN: 0168-9452 in der Anmeldung erwähnt
- JIA YULIN ET AL: "Rapid transcript accumulation of pathogenesis-related genes during an incompatible interaction in bacterial speck disease-resistant tomato plants." PLANT MOLECULAR BIOLOGY, Bd. 40, Nr. 3, Juni 1999 (1999-06), Seiten 455-465, XP002159399 ISSN: 0167-4412

## Beschreibung

Die vorliegende Erfindung betrifft eine isolierte Nukleotid-Sequenz mit der Eigenschaft eines Promotors zur Erhöhung der Abwehrreaktion einer Pflanze gegen Pathogenbefall und Genkonstrukte mit einer solchen Nukleotid-Sequenz. Die Erfindung betrifft weiterhin ein Verfahren zur Erhöhung der Abwehrreaktion einer Pflanze gegen Pathogenbefall sowie transgene Pflanzen und Samen dieser Pflanzen.

Es ist bekannt, daß Pflanzen von verschiedenen Erregern und Parasitenarten befallen werden. Dabei sind Kulturpflanzen meist anfälliger für einen Parasitenbefall als ihre wildwachsenden Verwandten.

Die Schäden an landwirtschaftlichen Kulturpflanzen durch Pathogenbefall führen jährlich zu Ernteausfällen von bis zu 50%. Eine besondere Bedeutung kommt dabei den Ausfällen durch Befall mit pilzlichen Pathogenen zu. Häufig trifft man Pilze an, bei denen es sich um chlorophyllfreie, heterotrophe Thallophyten handelt. Ihre Thalli sind oft fädig und in der Regel von einer Zellwand umgeben.

Da der Einsatz von Fungiziden nur begrenzt möglich ist, ist es wünschenswert, Resistenzen gegen pilzliche Pathogene auch in Kulturpflanzen zu verwirklichen. Die Züchtung toleranter/resistenter Kulturpflanzen ist daher von besonderer wirtschaftlicher Bedeutung. Traditionelle Züchtungsprogramme sind jedoch in den Möglichkeiten, fremde Gene einzukreuzen, stark limitiert, zudem ist der zeitliche und personelle Aufwand sehr hoch.

Die Entwicklungen der Pflanzenbiotechnologie erlauben es, in einen grossen Teil der bekannten Nutzpflanzen Gene einzubringen und die von den Genen kodierten Proteine zu exprimieren.

Für die Expression eines Gens kommt der Auswahl eines geeigneten Promotors eine erhebliche Bedeutung zu. Infolgedessen existiert ein großes Bedürfnis nach gut charakterisierten Promotoren mit spezifischen Eigenschaften.

Promotoren können in Pflanzen eine konstitutive, eine organ-, gewebe- bzw. zellspezifische oder eine induzierbare Aktivität besitzen. Konstitutive Promotoren sind in der Lage, während der normalen Entwicklung einer Pflanze einen mit ihnen verbundenen kodierenden Bereich in allen Geweben zu exprimieren. Das Expressionsniveau muß dabei nicht für alle Gewebe und alle Zelltypen gleich groß sein. Konstitutive Promotoren können pflanzlichen, bakteriellen oder viralen Ursprungs sein.

Eine große Zahl pflanzlicher Promotoren konnte in den letzten Jahren isoliert und auf ihre Wirkung hin untersucht werden. Breite Anwendung haben inzwischen die aus *Agrobacterium tumefaciens* isolierten Octopinsynthase (ocs), Nopalinsynthase (nos) und Mannopinsynthase (mas) bzw. TR-Promotoren (De Greve et al., 1982, Depicker et al., 1982; Velten et al., 1984) und der 35S-Promotor des Blumenkohlmosaikvirus (Odell et al., 1985) gefunden. Pflanzliche Promotoren mit einer konstitutiven Aktivität sind für Tabak (WO 97/28268) und Himbeere (WO 97/27307) beschrieben worden.

Organ-, gewebe- oder zeitspezifische Promotoren können für die Expression von Genen in spezifischen Pflanzenteilen verwendet werden. Spezifität kann in diesem Zusammenhang bedeuten, daß ein Promotor hauptsächlich oder ausschliesslich in einem Organ, Gewebe oder Zelltyp aktiv ist. Hauptsächlich in einem bestimmten Organ aktiv sind z.B. die Tomatenpromotoren TFM7 und TFM9 in Tomatenfrüchten (US 5,608,150), ein Rapspromotor in Wurzeln (WO 94/02619), ein Sonnenblumenpromotor in Samen (WO 98/45460) und ein Kartoffelpromotor (WO 98/18940) in Blättern. Diese genannten Promotoren zeigen ihre höchste Aktivität in den erwähnten Organen. Eine exklusive, ausschließliche Aktivität für ein bestimmtes Kompartiment wurde für einen schließzellspezifischen Promotor aus der Kartoffel (DE 42 07358 A1), für den tapetum-spezifischen Promotor TA29 aus Tabak (EP 0344 029 B1) und für den pistil- und pollen-spezifischen SLG Promotor aus Brassica (Dzelzkàlns et al., 1993) beschrieben.

Induzierbare Promotoren sind regulatorische Elemente, deren Aktivität durch endogene oder exogene Reize reguliert wird. Endogene Reize können entwicklungsabhängige Vorgänge, Phytohormone oder kritische Metabolitkonzentrationen sein. Exogene Stimuli, die auf eine Pflanze einwirken und die einen Promotor aktivieren können, sind Licht, abiotischer und biotischer Streß. Zu den abiotischen Streßfaktoren zählen Trockenheit, Kälte, Hitze und hohe Salzkonzentrationen im Erdboden oder die mechanische Verletzung von Pflanzentellen. Biotische Streßsituationen in Pflanzen werden durch Pathogenbefall hervorgerufen.

Entwicklungsabhängig regulierte Promotoren steuern die Entwicklung und Reife von Organen oder ganzen Pflanzen. Der Übergang von grünen, photosyntetisch aktiven Blättern zu seneszenten Blättern ist u.a. charakterisiert durch die Reduktion der Promotoraktivität von Genen, die an der Photosynthese beteiligt sind. Dazu gehören die Promotoren das Chlorophyll a/b Bindungsproteinsgens (cab) und des Gens der kleinen Untereinheit der Ribulose-1,6-bisphosphatsynthase (ssu). Gleichzeitig kommt es mit dem Einsetzen der Seneszenz zur Aktivierung von seneszenz-speziflschen Promotoren.

Darüber hinaus ist ein organspezifischer Promotor bekannt, der im Speicherwurzelgewebe der Zuckerrübe aktiv ist (WO 97/32027). Ein pathogenaktiver Promotor ist ferner in WO 92/17591 beschrieben.

Pathogen responsive Promotoren sind schließlich auch aus Rushton und Somssich, 1998, bekannt. Diese Druckschrift beschreibt das Vorhandensein von W-Boxen. Allerdings enthält diese Druckschrift keine Angabe über eine reverse, komplementäre Anordnung von W-Boxen.

Aufgabe der vorliegenden Erfindung ist es, einen weiteren Pathogen-induzierbaren Promotor anzugeben, mit dessen Hilfe eine verbesserte Pathogenabwehr einer Pflanze möglich ist.

Erfindungsgemäß erfolgt die Lösung der gestellten Aufgaben durch eine isolierte Nukleotid-Sequenz, die mindestens zwei verschiedene cis-Elemente, ausgewählt aus der Gruppe der cis-Elemente
a) L-Box oder L-Box ähnliche Sequenz, welche die Hexanukleotid-Sequenz CCTAc/aC enthält und in mindestens einem weiteren Nukleotid mit der L-Box übereinstimmt,
b) GCC-Box
c) W-Box mit einer Hexanukleotidsequenz TTGACC
enthält, wobei eine erste W-Box mit der Sequenz TTGACC in normaler und eine zweite W-Box in reverser, komplementärer Orientierung vorliegt.

In den Unteransprüchen sind bevorzugte Ausführungsformen der Erfindung angegeben.

Zunächst werden einige in der Anmeldung verwendeten Begriffe näher erläutert, um klarzustellen, wie sie hier verstanden werden sollen.

Unter Promotor ist eine DNA-Sequenz zu verstehen, die die Expression eines Gens unter seiner Kontrolle in Abhängigkeit von endogenen und exogenen Faktoren steuert. Zu diesen Faktoren gehören z.B. induktoren, Repressoren und ähnliche DNA-bindende Proteine, als auch Umwelteinflüsse. Ein Promotor kann aus mehreren Elementen bestehen. Er umfaßt jedoch mindestens ein regulatorisches Element, das für die Transkription des unter seiner Kontrolle stehenden Gens zuständig ist.

Derivate einer Nukleotid-Sequenz sind verkürzte Versionen dieser Sequenz mit gleichen, modifizierten oder singulären Eigenschaften wie die Ausgangssequenz.

Elizitoren sind Substanzen, die z.T. aus dem Pathogen stammen, z.T. werden sie durch Pathogene aus der Pflanzenzellwand freigesetzt. Elizitoren aus Pilzen sind einerseits neutrale, verzweigte Glucane, andererseits Glycoproteide. Elizitoren aus der Zellwand dürften Fragmente von Pectinstoffen sein. Elizitoren können innerhalb von 20 min auf Transkriptionsebene Genexpression auslösen, wie am Beispiel der Synthese von PALspezifischer mRNA in Petersilien-Zellkulturen gezeigt wurde.

Pathogeninduzierbarkeit bedeutet das Einwirken von äußeren Faktoren auf eine Pflanze, die eine Abwehrreaktion derselben nach sich zieht. Dabei kann es sich um Angriffe durch Insekten (Fraß), Bakterien, Pilze oder andere Pathogene handeln, aber auch um abiotische Einwirkungen, wie mechanische Verwundungen (z.B. durch Hagelschlag).

Die Pathogeninduzierbarkeit des hier beschriebenen Promotors bedeutet die erhöhte Transkription des Gens, das unter der Kontrolle des Promotors steht, wenn der pathogene Induktor vorliegt und welches sich folglich in einer aktiven Abwehrreaktion der befallenen Pflanze ausdrückt.

Direkte antifungale Wirkung bedeutet, daß Genprodukte unmittelbar antifungal wirken, indem sie z.B. Zellwände auflösen oder für Phytoalexinsynthasen codieren bzw. für Metabolite, die hemmend in den pilzlichen Stoffwechsel eingreifen.

Indirekte antifungale Wirkung bedeutet, daß Genprodukte die pflanzliche Genabwehr aktivieren. Zu diesen Genen gehören z.B. Resistenzgene, Komponenten der Signaltransduktion (wie Kinasen, Phosphatasen), Transkriptionsfaktoren oder Enzyme, die Signalsubstanzen produzieren (wie Ethylenbildende, Salicylsäurebildende oder Jasmonatbildende Enzyme, reaktive Sauerstoffspezies bildende Enzyme, Stickstoffmonoxydbildende Enzyme).

Unter Altersabhängigkeit bzw. Entwicklungsabhängigkeit ist die unterschiedliche Intensität der Promotoraktivierung und damit der Expression des vom Promotor kontrollierten Gens je nach Pflanzenalter zu verstehen.

Als "sink"-Blätter werden hier solche Blätter bezeichnet, die aufgrund ihrer geringen Größe mehr Kohlenhydrate verbrauchen als sie selber produzieren.

"Source"-Bätter dagegen sind Blätter, die aufgrund ihrer Größe mehr Kohlenhydrate produzieren als sie selber verbrauchen.

Unter Infektion ist der früheste Zeitpunkt zu verstehen, bei dem der Stoffwechsel des Pilzes (bzw. das Wachstum des Pilzes) auf eine Penetration des Wirtsgewebes vorbereitet wird. Dazu gehören z.B. das Auswachsen von Hyphen oder die Bildung von spezifischen Infektionsstrukturen wie Penetrationshyphen und Appressorien.

Unter Inokulation ist das physikalische Zusammentreffen von Pathogen und Wirt zu verstehen.

Die Erfindung wird nachfolgend und mit Bezug auf die Figuren und Beispiele näher erläutert:

Die erfindungsgemäße Nukleotid-Sequenz enthält mindestens zwei verschiedene cis-Elemente, ausgewählt aus der folgenden Gruppe
a) L-Box oder L-Box ähnliche Sequenz, welche die Hexanucleotid-Sequenz CCTAc/aC enthält in mindestens einem weiteren Nukleotid mit der L-Box übereinstimmt,
b) GCC-Box mit der Kernsequenz GCCGCC
c) W-Box mit der Hexanukleotidsequenz TTGACC,
wobei eine erste W-Box in normaler und eine zweite W-Box in reverser, komplementärer Orientierung vorliegt.

Unter L-Box wird eine 12 bp umfassende Sequenz t/aCTc/aACCTAc/aCc/a (Lois et al., 1989; da Costa e Silva et al., 1993) verstanden wobei t/a bzw. c/a bedeutet, daß ein T oder A bzw. C oder A an dieser Stelle in der Sequenz vorhanden sein kann. Als L-Box ähnliche Sequenz wird eine Sequenz verstanden, die die Hexanukleotidkernsequenz CCTAc/aC enthält und in mindestens einem weiteren Nukleotid mit der L-Box übereinstimmt. Die L-Box bzw. L-Box ähnlichen Sequenzen sind in den Promotoren der Phenylalanin-Ammonium-Lyase-(pal)-Gene aus Petersilie, Bohne und Arabidopsis thaliana und einiger weiterer Gene des Phenylpropan-Stoffwechselweges (Lois et al., 1989; Ohl et al., 1990) identifiziert worden. Die L-Box wurde durch die "in vivo DNA footprinting" Technik als ein Ort einer UV-Licht und elizitor- induzierbaren DNA/Protein-Interaktion identifiziert. Die Kernsequenz CCTAc/aC ist der Sequenzbereich an dem die stärkste Hypomethylierung auftritt (Lois et al., 1989). Das Auftreten des Box L sowie des Box P "footprints" fällt zeitlich zusammen mit dem Beginn der Transkription des pal-Gens nach UV- bzw. Elizitorstimulation (da Costa e Silva et al., 1993).

Unter GCC-Box wird eine 11 bp große Sequenz TAAGAGCCGCC (Ohme-Takagi and Shinshi, 1995) verstanden. Als GCC- Box wird auch eine Nukleotidsequenz verstanden, die nur das Kernmotiv GCCGCC der GCC-Box enthält. Die GCC-Box vermittelt die Aktivierung von Promotoren durch die Signalsubstanz Ethylen und wurde für die Promotoren einiger Pathogenabwehrgene beschrieben (Ohme-Takagi and Shinshi, 1995).

Unter W-Box wird eine Hexanukleotidsequenz TTGACC verstanden. W-Boxen sind auch in den Promotoren anderer Pathogenabwehrgene beschrieben worden (Rushton and Somssich, 1998). Die Bedeutung dieses cis-Elementes für die Pathogen-Induzierbarkeit der Promotoren wurde für das Maisgen PRms (Raventös et al., 1995) und die Petersiliengene PR1-1 und PR1-2 nachgewiesen (Rushton et al., 1996).

Weitere Kombinationen von cis-Elementen sind in den Unteransprüchen 2 bis 8 beschrieben.

In einer bevorzugten Ausführungsform der Erfindung enthält die Nukleotid-Sequenz ein weiteres cis-Element, das für eine Induktion der Abwehrreaktion durch Salicylsäure verantwortlich ist. Dabei handelt es sich um ein Fragment, das ein SAR-Element (Salicylic acid response element) ist. Das SAR-Element befindet sich vorzugsweise in reverser komplementärer Orientierung in direkter Nähe zu einer W-Box.

Ein SAR-Element mit der Sequenz TTCGACCTCC wurde als Kernsequenz eines 76 bp großen DNA Fragments des PR2-d Gens aus Tabak identifiziert (Shah and Klessig, 1996). Das 76 bp große Fragment mit dem SAR-Element ist verantwortlich für die Salicylsäure-Induzierbarkeit des PR2-d Gens. SAR-Elemente im Sinne dieser Anmeldung sind TTCGACCTCC-Sequenzen einschließlich entsprechender Konsensus-Sequenzen. Ein SAR-Element ist auch die Sequenz TTCGACCTCG. Diese liegt bevorzugt in reverser, komplementärer Orientierung zum Transkriptionsstart bei Position 4176-4167 und damit nur 2 bp neben der dritten W-Box.

Weitere Ausführungsformen der Erfindung können besondere Abfolgen und Kombinationen der cis-Elemente vorsehen. Bevorzugt ist eine enge Kopplung des 10 bp großen SAR-Elementes an eine W-Box. Möglicherweise ist die enge Kopplung für eine verstärkte Salicylsäure-Induzierbarkeit des Promotors verantwortlich. Ebenso scheint das Vorliegen dreier L-Box ähnlicher Sequenzen vorteilhaft für die Pathogeninduzierbarkeit einer Nukleotid-Sequenz zu sein.

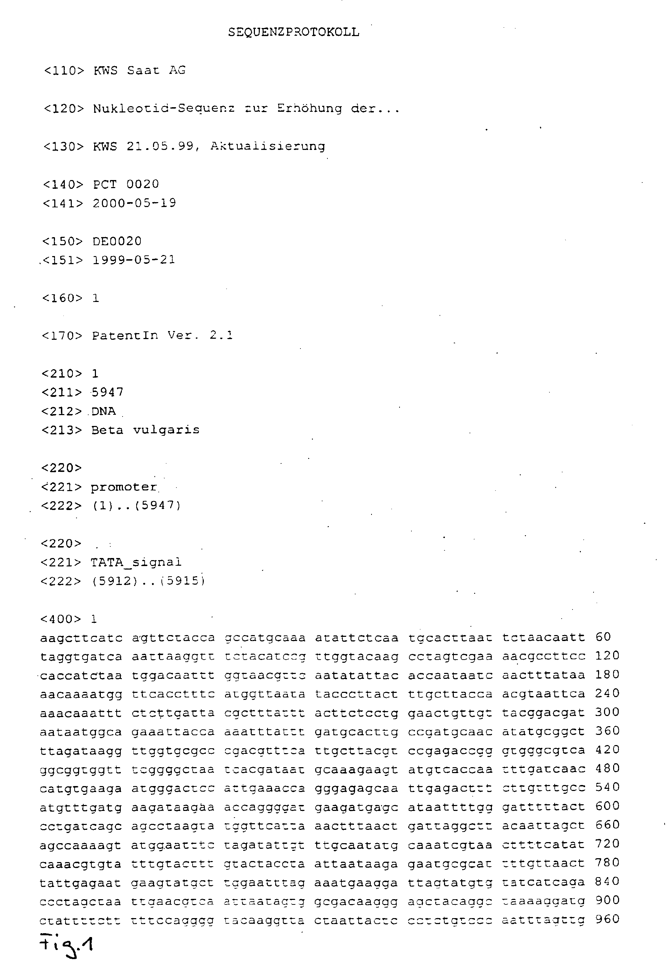

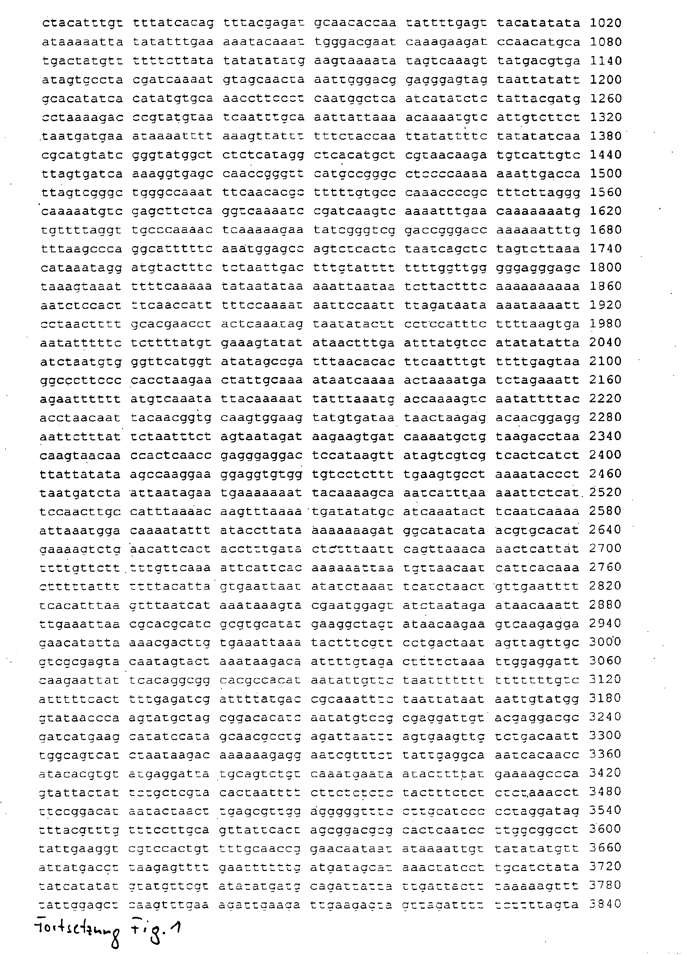

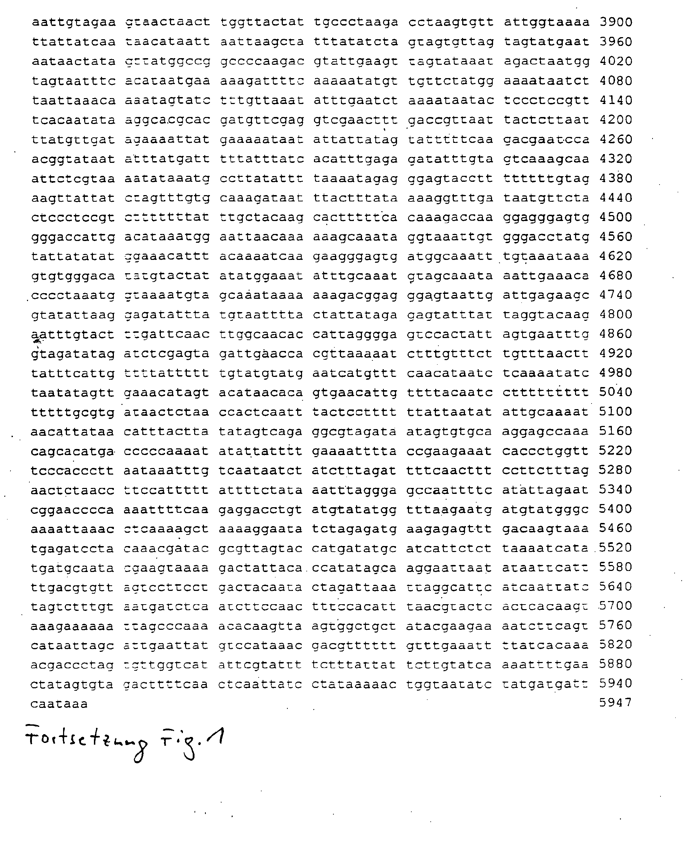

Eine Lösung der eingangs gestellten Aufgabe stellt insbesondere die Nukleotid-Sequenz (SEQ ID No.1) gemäß Fig. 1 dar. Diese Sequenz und hiervon abgeleitete Sequenzen sind geeignete Promotoren mit einer guten Pathogeninduzierbarkeit. Abgeleitete Sequenzen sind u.a. homologe Sequenzen mit einer Homologie von mind. 50%, wobei die Homologie im wesentlichen auf Übereinstimmungen im Bereich der cis-Elemente zurückzuführen ist.

Der Ausdruck "Homologie" bedeutet hierbei Homologie auf DNA-Ebene, die gemäß bekannter Verfahren, z.B. der computergestützten Sequenzvergleiche (S.F. Altschul et al. (1990), Basic Local Alignment search tool, J.Mol. Biol. 215: 403-410) bestimmt werden kann.

Es können auch Derivate einer Nukleotid-Sequenz nach Figur 1 hergestellt werden. Ausgehend von einer Sequenz gemäß Fig. 1 erfolgt hierzu eine Polymerasekettenreaktion mit einem der folgenden Primerpaare
a) P0/P4480
b) P0/P4047
c) P0/P3017
d) P0/P2661
e) P0/P2339
f) P0/P1889
g) P0/P1777
h) P0/P1777*
i) P0/P814
j) P0/P368,
wobei
P0 mit der Sequenz ACT GAC CAC CCG GGG TGG ATT TAT TG
P4480 mit der Sequenz CCG GGT CGA CGC CGG GCC TCC CCA AA
P4047 mit der Sequenz TCC AAT TGT CGA CAA TAA AAT TC
P3017 mit der Sequenz TAT AAC AAG AAG TCG ACA GGA GAA CAT ATT
P2661 mit der Sequenz GTG AAG TCG ACT GAC AAT TTT GGC AGT CAT C
P2339 mit der Sequenz TTA TTG AAG GTC GAC CAC TGT TTT GCA ACC
P1889 mit der Sequenz AAT ATG TTG ACC TAT GGA AAA TAA TC
P1777 mit der Sequenz GTT CGA GGT CGA CCT TTG ACC GTT AAT TAC
P1777* mit der Sequenz GTT CGA GGT CGA CCT TAG ACT GTT AAT TAC
P814 mit der Sequenz AGT CAG AGG CGT CGA CAA TAG TGT GC und
P368 mit der Sequenz TAT AAT TCA TGT TGA CGT GTT AGT CCT TCC
oder einer jeweils hiervon abgeleiteten Sequenz bindet.

Darüber hinaus läßt sich auch eine Sekretion exprimierter Proteine in Apoplasten erreichen, indem man die Nukleotid-Sequenz transkriptionell mit der Signalsequenz des apoplastischen Invertase-Inhibitors aus Tabak fusioniert.

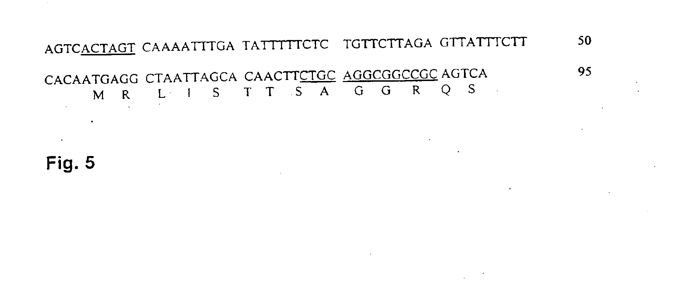

Nukleotid-Sequenzen gemäß dieser Erfindung können aber auch mit einer Nukleotid-Sequenz am 3'-Bereich translationell erweitert werden. Bevorzugt läßt sich eine translationelle Fusion mit Hilfe einer Nukleotid-Sequenz aus der Sequenz nach Fig. 5 durchführen.

Entsprechend der dargestellten Erfindung lassen sich transgene Pflanzen herstellen. Durch die Einschleusung der Nukleotid-Sequenzen in die zu verändernden Pflanzen kann Pathogenresistenz beeinflußt werden. Es sind keine pleiotropen Geneffekte zu erwarten. Damit bleibt die Leistungsfähigkeit des Zuchtmaterials unberührt. Die resultierenden transgenen Pflanzen zeigen eine erhöhte Resistenz gegenüber Pathogenen, vor allem gegenüber pilzlichen Pathogenen. Diese können aus der Gruppe umfassend Plasmodiophoromycetes, Oomycetes, Ascomycetes, Chytridiomycetes, Zygomycetes, Basidiomycetes, Deuteromycetes stammen.

Mit den erfindungsgemäßen Nukleotid-Sequenzen lassen sich insbesondere Genkonstrukte herstellen, bei denen das Gen ein Pathogenabwehrgen ist. Vorteilhafterweise ist das Gen ein Resistenzgen oder ein Avirulenzgen.

Nach einer weiter bevorzugten Ausgestaltung der Erfindung zeigt das Gen direkte oder indirekte antifungale Wirkung.

Beispielsweise läßt sich ein mit einer Signalsequenz versehener Promotor aus pAnjasek mit dem T4-Lysozym-Gen fusionieren, das Konstrukt in Pflanzen transformieren, welche dann eine erhöhte Pathogenabwehrreaktion, speziell gegenüber pilzlichen Pathogenen, aufweisen.

Die neuen Nukleodid-Sequenzen zeichnet sich weiterhin dadurch aus, daß sie in verschiedenen Organen und Geweben transgener Pflanzen, wie Blättern, Sprossen und/oder Wurzeln, nach Pathogenbefall oder Verwundung zur schnellen lokalen Expression des unter ihrer Kontrolle stehenden Gens führen. Diese Eigenschaft macht diese Sequenzen zu einem idealen Regulationselement zur kontrollierten Expression von antifungalen Verbindungen und damit zur Entwicklung von pilzresistenten Pflanzen. Entsprechende Vorteile ergeben sich auch bei den von den Nukleotid-Sequenzen in oben beschriebener Form abgeleiteten Derivaten.

Die erfindungsgemäßen Nukleotid-Sequenzen sind auch in Seitenwurzeln, der Pfahlwurzel und den Petiolen von Zuckerrüben konstitutiv aktiv. In Blättern von Zuckerrüben zeigen diese Sequenzen, unabhängig vom "sink" oder "source" Charakter der Blätter, eine altersabhängige, insbesondere verstärkt altersabhängige Aktivitätszunahme. Die neuen Nukleotidsequenzen zeichnen sich zudem dadurch aus, daß sie in Blättern transgener Pflanzen mit zunehmenden Pflanzenalter eine steigende Expression des unter ihrer Kontrolle stehenden Gens erlauben. Hierdurch lassen sich ausgezeichnet Pilzerkrankungen von Pflanzen, die wie die Zuckerrübe eine Altersanfälligkeit gegenüber dem Pathogen *Cercospora beticola* zeigen, bekämpfen.

Diese überraschende Eigenschaft einer altersabhängigen Expression kann z.B. in südlichen Ländern eine Lösung für Folgeprobleme einer Pilzinfektion darstellen. Z.B. werden in Italien, wo der Zuckerrübenanbau unter starken Cercospora-Infektionen zu leiden hat, frühe und mittelfrühe Sorten derzeit bereits im Juli/August geerntet, um einen Ausfall durch Pathogenbefall zu umgehen. Hier bietet ein in der vorliegenden Anmeldung dargestellter Promotor die Lösung, daß er zum Zeitpunkt des höchsten Infektionsdrucks (Juli/August) aktiviert werden kann. Somit kann bei diesen Befallssituationen auch der Anbau von spätreifen Sorten mit transgener Pathogenresistenz ermöglicht werden.

Weiterhin kann die Aktivitätszunahme eines solchen Promotors in Blättern aber auch genutzt werden, um sowohl Qualitäts- als auch altersabhängige Verarbeitungseigenschaften von Pflanzen zu verbessern. Dabei kommen beispielsweise Eigenschaften wie der Verringerung von Lagerungsverlusten bei gelagerten Rüben, oder einer Verminderung der Bildung von schädlichem Stickstoff durch antisense-Expression eines Transportergens Bedeutung zu.

Tabelle 2 zeigt die überraschend stark auftretende Elizitor-Induzierbarkeit des Promotors. Im Vergleich zur Kontrolle, der nicht-transgenen Rapssorte Drakkar, zeigen die beiden transgenen Linien einen Induktionsfaktor von 17,7 bzw. 16,9 nach Eliziterierung.

Für die Elizitor-Induzierbarkeit läßt sich als Elizitor das Enzym Polygalacturonase aus *Rhizopus* einsetzen.

Durch die Nukleotid-Sequnzen gemäß der Erfindung ist die Induktion der Abwehrreaktion gegenüber pilzlichen Pathogenen bereits ab dem ersten Tag nach Inokulation nachweisbar ist. Diese überraschende Eigenschaft ist vor allem für die Frühphase der Infektion relevant, da eine Infektion durch *Cercospora beticola* erst vier Tage nach der Inokulation beginnt.

In seiner natürlichen Umgebung in Zuckerrüben zeigt der neue Promotor während den ersten 12 Wochen der Pflanzenentwicklung sowohl in "sink" als auch in "source" Blättern eine geringe, aber stetig zunehmende Aktivität, die aber noch nicht größer ist als die Aktivität in den übrigen Organen (Petiole, Pfahl-, Seitenwurzel). Nach 12 Wochen kommt es in nichtinfizierten "sink" und "source" Blättern zu einem Anstieg der Promotoraktivität, die die Promotoraktivität in den übrigen Organen weit übersteigt. Dies weist auf eine erhöhte Altersaktivität des hier beschriebenen Promotors in Blättern hin.

Der Promotor wird sowohl in Rübenblättern durch Blattpilze wie z.B. *Cercospora beticola* als auch in Rübenwurzeln durch wurzelbürtige Schaderreger wie z.B. *Rhizoctonia solani* aktiviert.

Die Aktivitätszunahme des Promotors in Blättern und dem Rübenkörper von Zuckerrüben korreliert mit dem Ausmaß des Pilzbefalls und der Gewebeschädigung. Durch die Verwendung des Promotors in Kombination mit einem geeigneten Gen kann aufgrund dieser Eigenschaft eine kurative Wirkung erzielt werden. Auch im Fall einer bereits erfolgten Infektion kann die weitere Ausbreitung eines Pathogens verhindert oder eingeschränkt werden.

In bevorzugter Weise wird ein Genkonstrukt bereitgestellt, das aus Gen und Promotor besteht, wobei das Gen unter der Kontrolle des Promotors steht. Dabei zeichnet sich das Gen dadurch aus, das es direkte oder indirekte antifungale Wirkung zeigt.

Mit Hilfe eines solchen Genkonstrukts läßt sich eine Erhöhung der Abwehrreaktion einer Pflanze gegen Pathogenbefall erreichen, wenn man das Genkonstrukt in dem Genom einer Pflanze stabil etabliert. Dabei kann das in Frage kommende Gen bereits in dem Genom der Pflanze vorhanden sein, wenn die erfindungsgemäße Nukleotid-Sequenz an entsprechender Stelle integriert werden kann.

Gemäß einer Ausgestaltung des Verfahrens der vorliegenden Erfindung läßt sich die Erhöhung der Abwehrreaktion durch biotische und/oder abiotische Faktoren auslösen. Als ein abiotischer Faktor kommt insbesondere Salicylsäure in Betracht.

Biotischer Faktor kann ein pflanzliches Pathogen, insbesondere ein pilzliches Pathogen, sein. Pilzliche Pathogen stammen vorzugsweise von einem Pilz, der ausgewählt ist aus der Gruppe umfassend Plasmodiophoromycetes, Oomycetres, Ascomycetes, Chytridiomycetes, Zygomycetes, Basidiomycetes, Deuteromycetes.

Dem Fachmann sind auch Verfahren bekannt, um aus Pflanzenzellen, die eine Nukleotid-Sequenz gemäß der vorliegenden Erfindung enthalten, Pflanzenteile und ganze Pflanzen zu regenerieren. Beispielsweise werden hierzu Verfahren beschrieben von Fennell et al., Plant Cell Rep. 11 (1992), 567-570; Stoeger et al. Plant Cell Rep. 14 (1995), 273-278; Jähne et al., Theor. Appl. Genet. 89 (1994), 525-533.

Zu den Pflanzen, denen aufgrund der vorliegenden Erfindng eine erhöhte Resistenz vermittelt werden kann, gehören praktisch alle Pflanzen. Insbesondere lassen sich Kulturpflanzen züchten, die eine Resistenz gegen pilzliche Pathogene aufweisen. Derartig resistente Kulturpflanzen sind ihren nicht resistenten Verwandten weit überlegen, da sie nicht von pilzlichen Pathogenen befallen werden können und daher weniger krankheitsanfällig sind. Weiterhin weisen derartig resistente Kulturpflanzen eine gleich hohe Qualität und Leistungsfähigkeit wie andere, nicht resistente Kulturpflanzen auf. Dies ist darauf zurückzuführen, daß mit Übertragung einer Nukleotid-Sequenz gemäß der vorliegenden Erfindung keine DNA-Sequenzen übertragen werden, die unter Umständen für unerwünschte Eigenschaften kodieren.

Geeignete Pflanzen im Sinne dieser Erfindung sind Nahrungsmittel und Rohstoffe liefernde Pflanzen, z.B. Kohlenhydrate liefernde Pflanzen (insbesondere Weizen, Mais, Reis, Roggen, Kartoffeln, Gerste, Hafer und Hirse), Öl und Fett liefernde Pflanzen (insbesondere Erdnuss, Ölpalme, Olive, Raps und Sonnenblumen), Zucker liefernde Pflanzen (insbesondere Zuckerrübe, Zuckerrohr, Zuckerhirse), Protein liefernde Pflanzen (insbesondere Erbsen, Bohnen, Kichererbsen, Linsen und Sojabohne), Fasern liefernde Pflanzen (insbesondere Baumwolle, Flachs, Hanf, Jute), Genussmittel liefernde Pflanzen (insbesondere Tabak, Tee- und Kakaosträucher), Holz liefernde Pflanzen (insbesondere Birken, Fichten, Tannen, Douglasien, Kiefern, Lärchen, Limba, Mahagoni, Buchen, Eichen, Zedern), Futtermittel liefernde Pflanzen (insbesondere Luzerne, und Futterrübe), Gemüse (insbesondere Gurken, Kohlarten, Kürbisse, Möhren, Paprika, Salate, Spinat, Radieschen und Tomaten), Obst (insbesondere Äpfel; Birnen, Kirschen, Melonen, Weintrauben, Citrus, Ananas und Bananen) weiterhin der Kautschukbaum und Zierpflanzen. Vorzugsweise ist die trangene Pflanze ausgewählt aus Zuckerrübe, Raps, Kartoffel, Mais, Sojabohne, Baumwolle, Weizen, Reis, Roggen, Gerste und Sonnenblumen. Diese Auswahl ist jedoch nicht einschränkend aufzufassen.

Die Erfindung betrifft aber auch transgene Pflanzen, die aus den erfindungsgemäß veränderten Pflanzen durch somatische Hybridisierung oder Kreuzung hervorgehen.

### BEISPIELE

Figur 1 zeigt die Nukleotid-Sequenz SEQ ID No. 1. Die Nukleotidsequenz ist in 5'-3' Orientierung dargestellt. Die Lage der allgemeinen Konsensus-Promotorsequenzen (CAAT - und TATA-Motive), der cis-Elemente (L-Box ähnliche Sequenz, GCC-Box, W-Box und SAR-Box) sind unten dargestellt. Die Nummerierung beginnt am 5'-Ende bei Pos. 1.

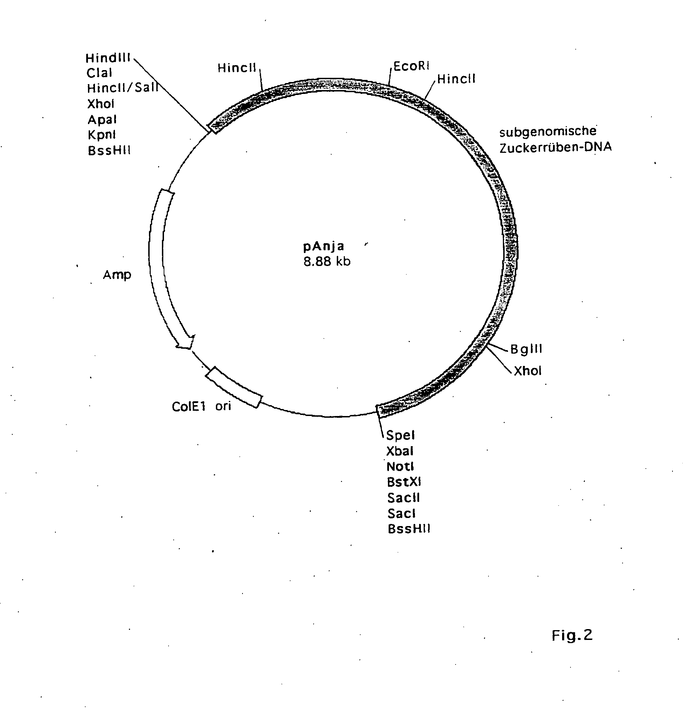

Figur 2 zeigt das Plasmid pAnja mit dem 5.947 kb großen genomischen DNA -Fragment aus Zuckerrübe (Beta vulgaris), das über *Hind*III und die gebluntete BamHI Restriktionsschnittstelle von pBluescriptII KS+ (Stratagene) subkloniert wurde. Neben den im Zuckerrübenfragment eingezeichneten Schnittstellen schneiden z.T. noch weitere Restriktionsenzyme des Polylinkers auch im klonierten Fragment.

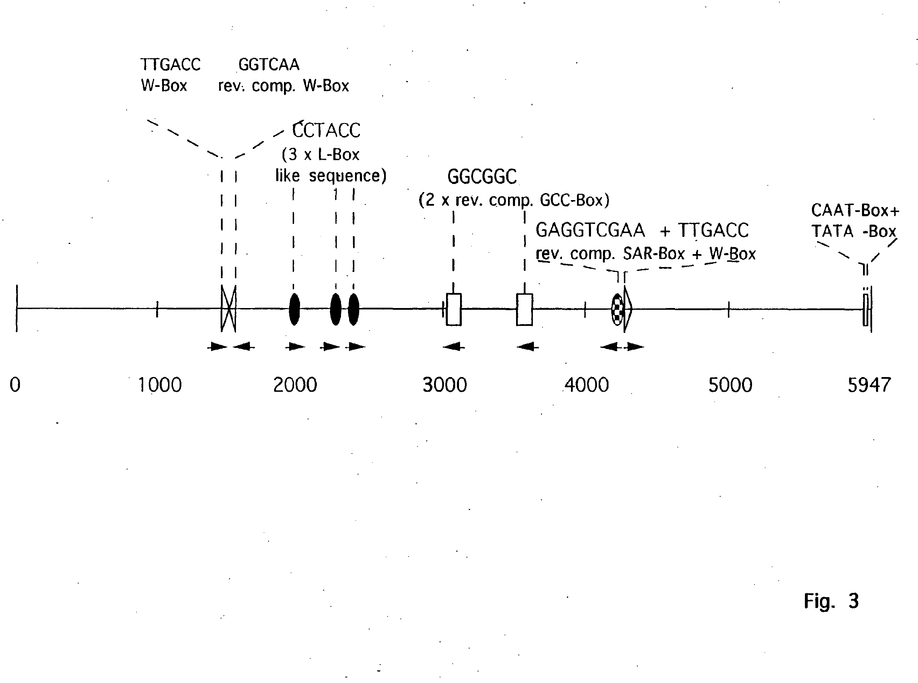

Figur 3 zeigt die Verteilung von cis-Elementen im Promotor. Die Pfeilspitzen geben jeweils die Orientierung der cis-Elemente, bezogen auf den Transkriptionsstart bei 5947 wieder.

Eine TATA-Box befindet sich bei Nukleotidposition 5912-5915 und eine CAAT-Box bei Nukleotidposition 5903-5906.

Es folgt bei Position 4179-4184 eine W-Box. Zwei weitere W-Boxen befinden sich an Position 1494-1499 sowie 1586-1581. Die W-Boxen bei den Positionen 4179-4184 und 1494-1499 liegen in normaler Orientierung mit Bezug auf die TATA-Box, die W-Box 1586-1581 liegt auf dem komplementären Strang in reverser Orientierung vor.

Nur 2 bp von der ersten W-Box bei 4179-4184 entfernt befindet sich eine SAR-Box bei Position 4176-4167 in reverser komplementärer Lage zur TATA-Box.

Eine GCC-Box liegt in zweifacher Wiederholung in reverser komplementärer Orientierung zur TATA-Box bei Position 3598-3593 sowie bei Position 3081-3076.

Eine L-Box ähnliche Sequenz liegt in dreifacher Wiederholung bei den Positionen 2331-2342, 2117-2228 und 1916-1927 vor. Alle drei L-Box ähnlichen Sequenzen liegen in gleicher Orientierung in bezug auf die TATA-Box.

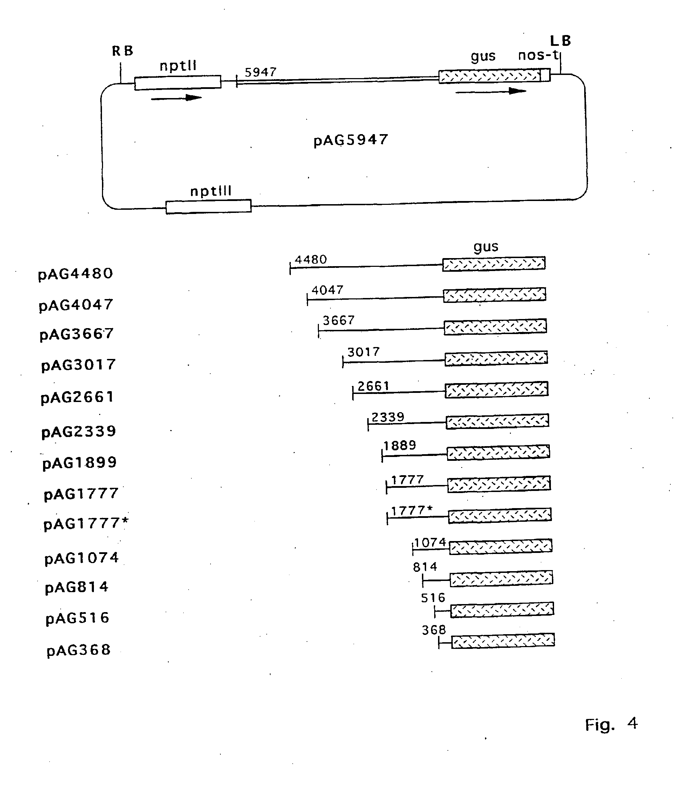

Figur 4 zeigt Promotordeletionen, ausgehend vom Vektor pAG5947. Die aufgeführten Plasmide resultieren aus transkriptioneller Kombination des Promotors mit einem gus-Gen (pAG4480, pAG4047, pAG3017, pAG2661, pAG2339, pAG1899, pAG1777, pAG1777*, pAG814 sowie pAG368) bzw. aus Restriktionsverdau (pAG3667, pAG1074, pAG516). Diese Promotordeletionen eignen sich für die Identifikation weiterer cis-Elemente.

Figur 5 zeigt die Nukleotid- und abgeleitete Aminosäuresequenz eines synthetischen DNA-Fragmentes. Das 95 bp große DNA-Fragment trägt am 5'-Ende die unterstrichene Erkennungsstelle für die Restriktionsendonuklease *Spe*I (ACTAGT) und am 3'-Ende die unterstrichenen Erkennungsstellen für die Restriktionsendonukleasen *Pst*I (CTGCAG) und *Not*I (GCGGCCGC). Die Nukleotidsequenz ist von Position 33-81 identisch mit den ersten 22 Nukleotiden des nichttranslatierten 5'-Bereichs und den ersten 27 Nukleotiden des codierenden Bereichs eines ß-1,3 Glucanase cDNA Klons aus der Zuckerrübe (Gottschalk and Mikkelsen, 1998).

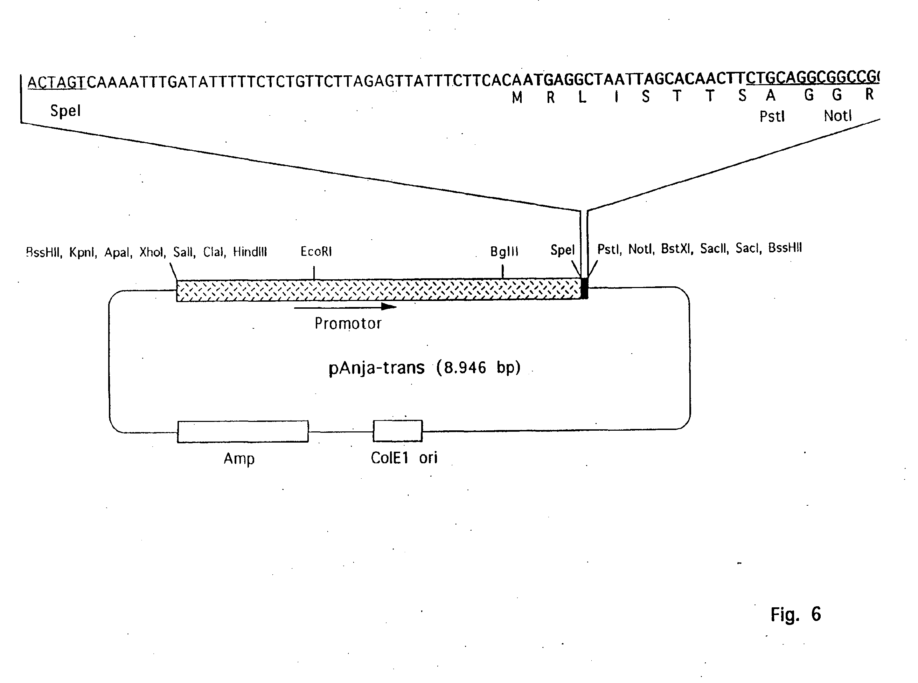

Figur 6 zeigt den 8.946 bp großen Vektor pAnja-trans. Durch Einführung eines synthetischen DNA-Fragments (vgl. Fig.5) hinter dem Transkriptionsstartpunkt des Promotors wird ein translatierbarer Bereich in den Vektor eingefügt. Der translatierbare Bereich codiert von Nukleotidposition 51-95 für den N-Terminus eines Proteins. Durch Nutzung der im 3'-Bereich des synthetischen Fragments gelegenen Erkennungsstellen für Restriktionsendonukleasen kann ein Fusionsprotein zwischen der 1.-12. Aminosäure eines synthetischen DNA-Fragmentes und eines beliebigen zu exprimierenden Gens erstellt werden.

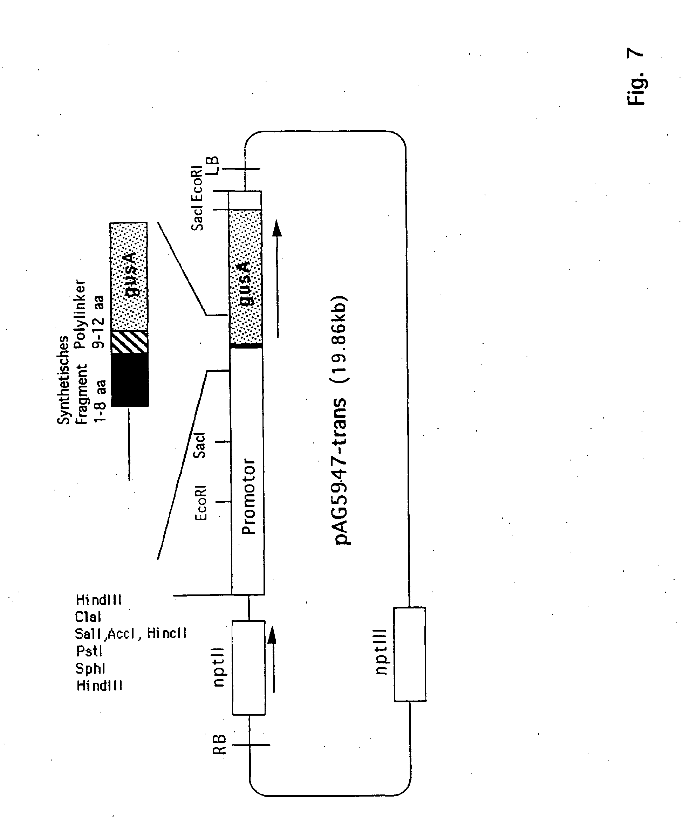

Figur 7 zeigt den 19,86 kb großen binären Pflanzentransformationsvektor pAG 5.947-trans. Innerhalb der von *Agrobakterium tumefaciens* zu übertragenden T-DNA (begrenzt durch die rechten (RB) und linken (LB) Borderbereiche) liegt der Selektionsmarker *npt*II und eine Reportergenkassette aus dem Promotor und dem gus-Gen. Durch Subklonierung des aus pAnja-trans stammenden Promotors liegt eine Translationsfusion zwischen dem synthetischen DNA-Fragment und dem gus-Gen vor.

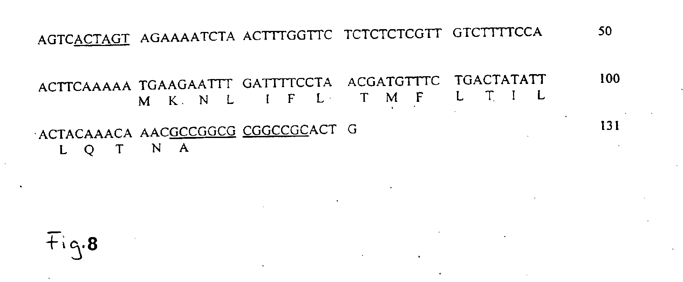

Figur 8 zeigt die Nukleotid- und Aminosäuresequenz der Signalsequenz des Invertase-Inhibitorgens (nt-inh1) aus Tabak nach Amplifikation durch die Primer Sek1 und Sek2. Der 5'-Bereich des cDNA Klons des nt-inh1 Gens (Greiner et al., 1998) entspricht der Nukleotidsequenz von 11-106. Die für die PCR-Amplifikation verwendeten Primer Sek1 und Sek2 entsprechen den Nukleotidsequenzen von Position 1-35 bzw. der komplementären Sequenz von 93-131. Die durch die PCR Reaktion zusätzlich an den 5'-und 3'-Bereich des cDNA Klons synthetisierten Nukleotidsequenzen mit den Erkennungsstellen für die Restriktionsenzyme *Spe*I (A/CTAGT), NaeI (GCC/GGC) und NotI (GC/GGCCGC) sind unterstrichen.

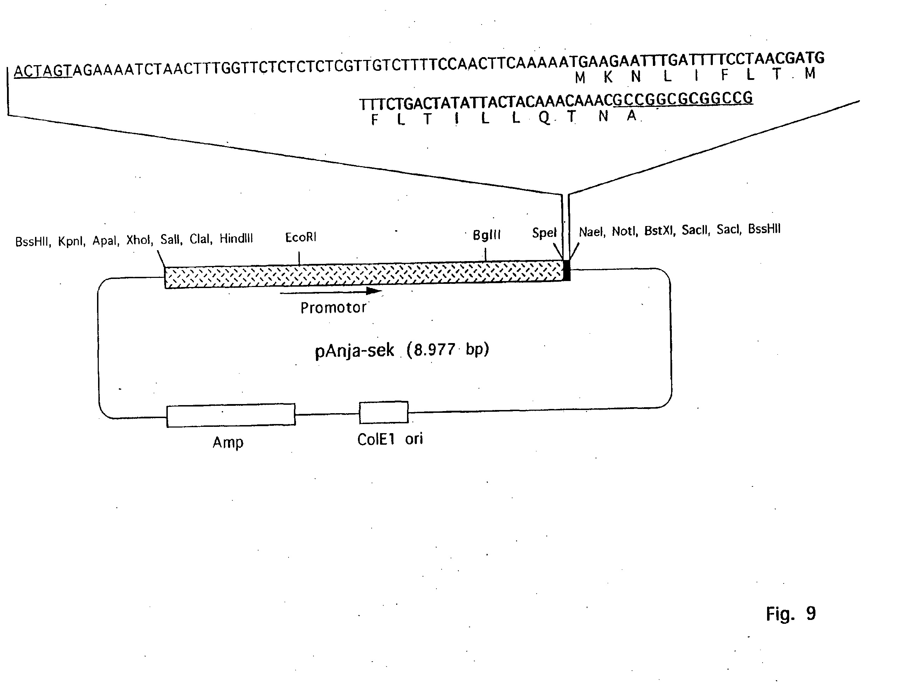

Figur 9 zeigt den 8.977 bp großen Vektor pAnja-sek. Durch Einführung eines synthetischen DNA-Fragments (vgl. Abb. 8) hinter den Transkriptionsstartpunkt des Promotors wird der 106 bp große 5'-Bereich des cDNA-Klons des Invertase-Inhibitors aus Tabak (Greiner et al. 1998) eingeführt. Dieser 5'-Bereich umfaßt die ersten 49 bp des transkribierenden, nicht translatierten Bereiches und die ersten 57 bp des codierten Bereiches. Durch Nutzung der *Nae*I Schnittstelle innerhalb des subklonierten PCR-Fragments kann ein beliebiges zu exprimierendes Gen mit der Signalsequenz des Invertase-Inhibitorgens fusioniert werden.

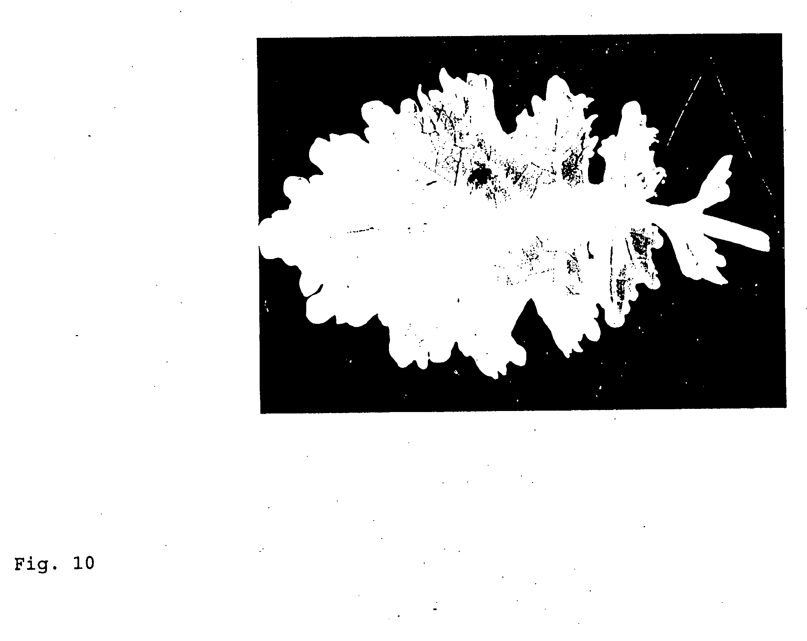

Figur 10 stellt den histochemischen Nachweis der lokalen Promotoraktivierung dar. Auf Blätter der Rapstransformante AG-5947-t49 wurden lokal 20µl Rhizopus Elizitor pipettiert, nach einer Inkubationszeit von 16h bei 24°C läßt sich die GUS-Aktivität histochemisch nachweisen. Die Blaufärbung des Gewebes zeigt die Bereiche an, in denen hohe GUS-Aktivität vorliegt.

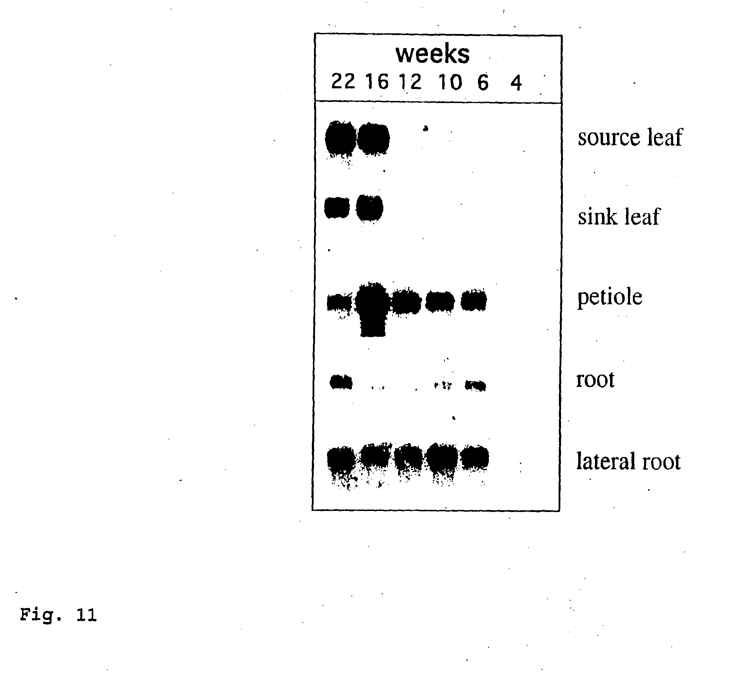

In Figur 11 wird die entwicklungsabhängige Promotoraktivität in einem RNA-Blot nachgewiesen. Dazu wurden je 10 µg Gesamtzell-RNA pro Organ (Lateralwurzel, Hauptwurzel, Petiole, "sink"-Blatt, "source"-Blatt) und Zeitpunkt (4, 6, 10, 12, 16 und 22 Wochen nach Aussaat) in einem denaturierenden Formaldehyd-Agarosegel aufgetrennt.

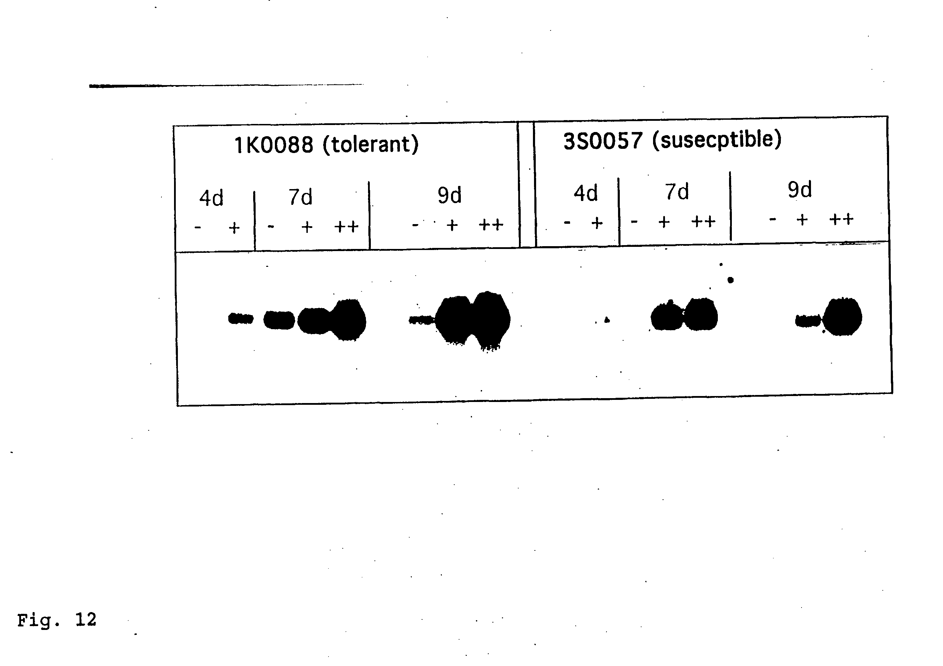

Figur 12 zeigt die Aktivierung des Promotors in Zuckerrüben nach Infektion mit *Cercospora beticola* unter Gewächshausbedingungen. Verwendet wurden Zuckerrüben des toleranten Genotyps 1K0088 und des anfälligen Genotyps 3S0057. Vier, sieben und neun Tage nach Inokulation wurden Blätter zur Isolierung von Gesamtzell-RNA geerntet. Die Aktivität des Promotors wurde durch RNA-Blot-Analyse bestimmt.

Die Schädigung der untersuchten Blütter durch Pilzbefall ist in der Abbildung symbolisch dargestellt: - gleich gesunde Kontrolle, + gleich schwach befallenes, ++ gleich stark befallenes.

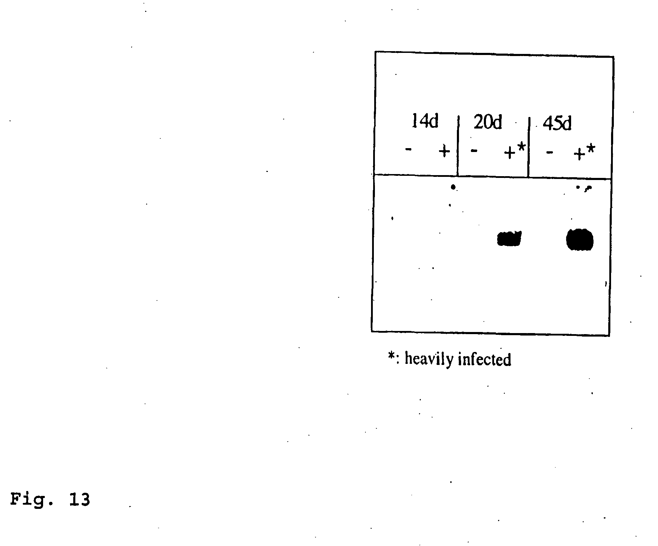

Figur 13 zeigt das Ergebnis der RNA-Blot-Analyse zur Messung der Transkriptbildung in Zuckerrüben des Genotyps 1K0088 nach Infektion mit dem Wurzelpathogen Rhizoctonia solani. Die Pilzinfektion erfolgte in einer Phytozelle, Proben zur Isolierung der Gesamtzell-RNA wurden jeweils 14, 20 und 45 Tage nach Inokulation genommen.

### Charakterisierung der Nukleotid-Sequenz nach Fig. 1

Die Nukleotid-Sequenz des 5947 bp grossen Promotorfragments ist in Fig. 1 wiedergegeben. Das Promotorfragment liegt subkloniert in dem Vektor pAnja (Fig. 2) vor. Der Vektor pAnja leitet sich von dem Plasmid pBluescript II KS+ (Stratagene) ab. Die Promotorsequenz ist charakterisiert durch mehrere cis-Elemente (Fig.3), deren Anwesenheit und Wiederholungen die Funktion des Promotors beeinflussen.
Die L-Box ähnliche Sequenz mit der Hexanukleotid-Sequenz CCTAAC liegt in drei Wiederholungen bei Position 2331-2342, 2117-2228 und 1916-1927 vor. Die L-Box ähnlichen Sequenzen haben alle die gleiche Orientierung in bezug auf den Transkriptionsstart und befinden sich innerhalb eines Sequenzbereichs von 420 bp.

Ein weiteres cis-Element mit der Nukleotidsequenz GCCGCC, die 'ethylene-responsive' oder GCC-Box, liegt in zweifacher Wiederholung in reverser, komplementärer Orientierung zur TATA Box bei Position 3598-3593 und Position 3081-3076 vor. Die GCC-Box vermittelt die Aktivierung von Promotoren durch die Signalsubstanz Ethylen und wurde für die Promotoren einiger Pathogenabwehrgene beschrieben (Ohme-Takagi and Shinshi, 1995).

Eine W-Box mit der Sequenz TTGACC liegt in dreifacher Wiederholung vor. Die erste W-Box erstreckt sich von Position 1494-1499, die zweite W-Box in reverser, komplementärer Orientierung von Position 1586-1581 und die dritte W-Box von Position 4179-4184. Charakteristisch ist auch die Anwesenheit der ersten zwei W-Boxen, eine in normaler und eine in reverser Orientierung innerhalb eines kurzen Sequenzabschnitts von 92 bp (1494-1586). Eine dritte W-Box (4179-4184) liegt in unmittelbarer Nähe zu einem weiteren, vierten cis-Element, dem SAR-Element.

Neben den cis-Elementen, die spezifische Eigenschaften des Promotors ausmachen, enthält der Promotor die grundlegenden Nukleotidmotive, die jeder Promotor für die Bindung der basalen Transkriptionsfaktoren benötigt. Zu diesen Motiven gehören eine CAAT-Box bei Nukleotidposition 5903-5906 und eine TATA-Box bei Nukleotidposition 5912-5915.

### Derivate der Nukleotid-Sequenz nach Fig. 1

Ausgehend von dem Vektor pAnja wurden durch PCR-Techniken bzw. der Nutzung singulärer Erkennungsstellen für Restriktionsendonukleasen Derivate des Promotors erzeugt. Die dafür notwendigen Klonierungsarbeiten erfolgten nach Sambrook et al., 1989. Diese Derivate unterscheiden sich vom 5947 bp großen Ausgangspromotor dadurch, daß sie kleiner als der Ausgangspromotor sind und nicht alle cis-Elemente aufweisen, die den Ausgangspromotor kennzeichen. Durch diese Deletionen weisen die Promotorfragmente ein neues Aktivitätsspektrum auf, die sie vom Ausgangspromotor unterscheiden. Weiterhin können durch diesen Deletionsansatz weitere, neue cis-Elemente identifiziert werden, die für die Promotoreigenschaften relevant sind.

Für die Erzeugung der Promotordeletionsfragmente durch PCR wurden jeweils zwei Oligo-Nukleotidprimer eingesetzt (Tabelle 1). Zum einen wurde der Primer P0 mit der Sequenz ACT GAC CAC CCG GGG TGG ATT TAT TG verwendet. Der Primer P0 bindet von Nukleotidposition 5941-5947 des Promotors und den angrenzenden Sequenzbereichen der Multiplen Klonierungsstelle des Vektors pBluescriptII KS+. Als zweiter Primer wurden die Oligonukleotide P4480, P4047, P3017, P2661, P2339, P1889, P1777, P1777*, P814 bzw. P368 verwendet, die an definierten Stellen innerhalb der Promotorsequenz binden. Der Primer P4480 mit der Sequenz CCG GGT CGA CGC CGG GCC TCC CCA AA bindet von Position 1464-1489. Der Primer P4047 mit der Sequenz TCC AAT TGT CGA CAA TAA AAT TC bindet von Position 1894-1921. Der Primer P3017 mit der Sequenz TAT AAC AAG AAG TCG ACA GGA GAA CAT ATT bindet von Position 2920-2949. Der Primer P2661 mit der Sequenz GTG AAG TCG ACT GAC AAT TTT GGC AGT CAT C bindet von Position 3282-3311. Der Primer P2339 mit der Sequenz TTA TTG AAG GTC GAC CAC TGT TTT GCA ACC bindet von Position 3600-3629. Der Primer P1889 mit der Sequenz AAT ATG TTG ACC TAT GGA AAA TAA TC bindet von Position 4054-4079. Die Primer P1777 und P1777* mit den Sequenz GTT CGA GGT CGA CCT TTG ACC GTT AAT TAC bzw. GTT CGA GGT CGA CCT TAG ACT GTT AAT TAC binden von Position 4164-4193. Der Primer P814 mit der Sequenz AGT CAG AGG CGT CGA CAA TAG TGT GC bindet von Position 5124-5194 und der Primer P368 mit der Sequenz TAT AAT TCA TGT CGA CGT GTT AGT CCT TCC bindet von Position 5570-5599.

Die PCR-Bedingungen lauten für die Primerpaare P0/P368, P0/P812, P0/P1777 und P0/1777* bei Verwendung von 1 ng des Plasmides pAnja, einer Primerkonzentration von 0.2 µM, 1.5 µ Taq-Polymerase (Amersham Pharmacia Biotech, Freiburg) und 25 µl Reaktionsvolumen in einen Multicycler PTC-200 (MJ Research, Watertown, USA) wie folgt:

| | | |
|---|---|---|
| 1 x | Schritt 1: 4 min | 95°C |
| 30 x | Schritt 2: 30 sec | 95°C |
| | Schritt 3: 30 sec | 57°C |
| | Schritt 4: 2 min | 72°C |
| 1 x | Schritt 5: 5 min | 72°C |

Für die Primerpaare P0/P4480, P0/P4047, P0/P3017, P0/P2661, P0/P2339 und P0/P1889 lauten die PCR-Bedingungen bei Verwendung von 10 ng des Plasmides pAnja, einer Primerkonzentration von 0.2 µM, 1.0 u Advantage KlenTaq-Polymerase-Mix (Clontech Laboratories, Heidelberg) und 25 µl Reaktionsvolumen in einen Multicycler PTC-200 (MJ Research, Watertown, USA) folgendermaßen:

| | | |
|---|---|---|
| 1 x | Schritt 1: 4 min | 95°C |
| 28 x | Schritt 2: 30 sec | 95°C |
| | Schritt 3: 30 sec | 57°C |
| | Schritt 4: 4 min | 72°C |
| 1 x | Schritt 5: 5 min | 72°C |

Die Anwendung dieser PCR-Bedingungen führt mit dem Primerpaar P0/P4480 zu einem 4503 bp großen DNA-Fragment. Mit Hilfe der Primerpaare P0/P4047, P0/P3017, P0/P2661, P0/P2339, P0/P1889, P0/P1777, P0/P1777*, P0/P814, P0/P368 werden 4073, 3047, 2685, 1913, 1800, 1800, 843 und 397 bp große DNA-Fragmente amplifiziert. Durch Einführung einer Erkennungsstelle für die Restriktionsendonuklease Smal (CCCGGG) in den Primer P0 und einer Erkennungsstelle für das Enzym *Sal*I (GTCGAC) in die Primer P4480, P4047, P3017, P2661, P2339, P1889, P1777, P1777*, P814 und P368 können die amplifizierten DNA-Fragmente mit den Enzymen Smal und Sal*I* nachgeschnitten und gerichtet in einen Vektor kloniert werden.

Das P0/P4480 Fragment wird als *Sma*I-*Sal*I Fragment in den mit Smal und *Sal*I geschnittenen Vektor pBluescriptII KS+ kloniert. Das resultierende Plasmid trägt aufgrund der Größe des Promotorfragmentes die Bezeichnung pA4480 (Tabelle 1). Entsprechend werden die PCR-Produkte P0/P4047, P0/P3017, P0/P2661, P0/P2339, P0/P1889, P0/P1777, P0/P1777*, P0/P814, P0/P368 mit Smal und *Sal*I geschnitten. Subkloniert in dem mit *Sma*I und *Sal*I geschnittenen Vektor pBluescriptII KS+ tragen die resultierenden Plasmide aufgrund der Promotorgröße die Bezeichnung pA4047, pA3017, pA2661, pA2339 und pA1889, pA1777, pA1777*, pA814 und pA368 (Tabelle 1).

### Transkriptionelle Kombination des Promotors mit einem Reportergen (gus-Gen)

Ausgehend von dem Plasmid pAnja wird der 5947 bp große Promoterbereich als transkriptionelle Fusion mit dem gus-Gen in dem Pflanzentransformationsvektor pBI101 (Laboratories, Heidelberg) kombiniert. Der resultierende Vektor trägt die Bezeichnung pAG5947 (Fig. 4). Dazu wird der Vektor pAnja zunächst mit dem Restriktionsenzym *Spe*I (Boehringer Mannhein) linearisert und die entstehenden DNA-Enden durch eine KlenowBehandlung aufgefüllt. Durch eine nachfolgende *Sal*I-Behandlung wird das 5947 bp große Promotorfragment freigesetzt und in den zuvor mit *Sal*I und Smal behandelten Vektor pBI101 inseriert. Entsprechend der beschriebenen Vorgehensweise werden die deletierten Promotorfragmente aus pA4480, pA4047, pA3017, pA2661, pA2339 und pA1889, pA1777, pA1777*, pA814 und pA368 in den Pflanzentransformationsvektor pBI101 kloniert. Die resultierenden Plasmide tragen die Bezeichnung pAG4480, pAG4047, pAG3017, pAG2661, pAG2339, pAG1889, pAG1777, pAG1777*, pAG814 und pAG368.

Drei weitere Promotordeletionen wurden ausgehend vom Vektor pAG durch die Verwendung von Restriktionsendonukleasen konstruiert. Der Vektor pAG wurde zum einem mit den Restriktionsenzym *Sal*I und partiell mit EcoRI, mit den Enzymen *Sal*I und *Xho*I und zum anderen mit den Enzymen *Sal*I und *Xba*I geschnitten. Durch diesen Restriktionsverdau wurde der Promotorbereich auf die Sequenz von Nukleotid 2280-5947, Position 4874-5947 und Position 5432-5947 verkürzt entfernt. Nach dem Auffüllen der Schnittstellen durch eine Klenowbehandlung konnten die Vektoren religiert und in *E.coli* transformiert werden.

Der verbleibende Promotoranteil ist nach der *Sal*I/*EcoR*I Behandlung 3667 bp, nach der *Sal*I/*Xho*I Behandlung 1074 bp und nach der *Sal*I/*Xba*I Behandlung 516 bp groß. Die entstehenden Vektoren tragen die Bezeichnung pAG3667, pAG1074 bzw. pAG516 (Fig. 4).

### Modifikation des Promotors für eine translationelle Fusion mit einem Reportergen (gus-Gen)

Um den beschriebenen Promotor nicht nur transkriptionell sondern auch translationell mit dem gus-Gen kombinieren zu können, wird ein synthetisches DNA-Fragment (Fig. 5) in den Vektor pAnja kloniert. Die 95 bp große Sequenz trägt am 5'-Ende die Erkennungsstelle für die Restriktiosendonuklease *Spe*I und am 3'-Ende die Erkennungsstellen für die Enzyme *Pst*I und *Not*I. Die Nukleotidsequenz kodiert von Nukleotidposition 51-95 für das 5'-Ende eines Proteins. Das Fragment aus Fig. 5 wird über die Spei und *Not*I Schnittstelle in den Vektor pAnja kloniert. Der resultierende Vektor trägt die Bezeichnung pAnja-trans (Fig. 6).

Für die Konstruktion der Translationsfusion mit dem gus-Gen wird der Vektor pAnja-trans mit dem Restriktionsenzym *Pst*I linearisiert und die überstehenden 3'-DNA-Enden werden durch T4-Polymerase-Behandlung in stumpfe Enden überführt. Der so behandelte Vektor wird nochmals mit dem Restriktionsenzym *Sal*I geschnitten und das 6014 bp grosse DNA-Fragment isoliert. Das Promotorfragment wird in den Pflanzentransformationsvektor pBI101, der zuvor mit den Restriktionsenzymen *Sal*I und *Sma*I geschnitten wurde, kloniert. Durch diese Klonierung erfährt des GUS-Enzym eine N-terminale Verlängerung von 12 Aminosäuren. Während die ersten 8 Aminosäuren von dem N-Terminus des synthetischen Fragmentes stammen, werden die Aminosäuren 9-12 von der Polyklonierungsstelle des Vektors pBI101 kodiert. Der so konstruierte Vektor trägt die Bezeichnung pAG5947-trans (Fig. 7). Entsprechende translationelle Fusionen lassen sich auch mit den Vektoren pA4480, pA4047, pA3017, pA2661, pA2339 und pA1889, pA1777, pA1777*, pA814 und pA368 herstellen, die anschließend nach Integration in den Pflanzentransformationsvektor pBI101 die Bezeichnungen pAG4480-trans, pAG4047-trans, pAG3017-trans, pAG2661-trans, pAG2339-trans, pAG1889-trans, pAG1777-trans, pAG1777*-trans, pAG814-trans und pAG368-trans tragen. Weiterhin werden ausgehend von dem Vektor pAG5947-trans in Anlehnung an die Konstruktion von pAG3667, pAG1074 und pAG516 durch Verwendung der Restriktionsenzyme *Sal*I und EcoRI, *Sal*I und *Xho*I bzw. *Sal*I und *Xba*I die Deletionskonstrukte pAG3667-trans, pAG1074-trans und pAG516-trans erzeugt.

### Modifikation des Promotors für eine Sekretion des exprimierten Proteins in den Apoplasten

Um mit Hilfe des Promotors auch heterologe Proteine im Apoplasten exprimieren zu können, wird der 5947 bp große Promotor transkriptionell mit der Signalsequenz des apoplastischen Invertase-Inhibitors aus Tabak (Greiner et al., 1998) fusioniert. Zu diesem Zweck wird der 106 bp große 5'-Bereich des Tabakinhibitors (Nt-inh1) mit Hilfe des Primer Sek1 (AGT CAC TAG TAG AAA'ATC TAA CTT TGG TCT CT) und des Primer Sek2 (CAG TGC GGC CGC GCC GGC GTT TGT TTG TAA TAT AGT CA) durch PCR aus dem Plasmid pGreiner amplifiziert. Die Primer Sek1 und Sek2 binden an Position 1-25 bzw. 83-106 des zu amplifizierenden 5'-Bereichs. Die gesamte Nukleotidsequenz des amplifizierten 131 bp großen Fragmentes ist in Fig. 8 dargestellt. Der amplifizierte 5'-Bereich umfaßt den 49 bp großen nichttranslatierten 5'-Bereich des cDNA-Klons und die ersten 57 bp des codierenden Bereichs. Durch die Primersequenz Sek1 wird an das 5'-Ende des PCR-Produktes zusätzlich eine Erkennungsstelle für die Restriktionsendonuklease *Spe*I (ACTAGT) und durch den Primer Sek2 an das 3'- Ende eine Erkennungsstelle für die Enzyme *Nae*I (GCCGGC) und *Not*I (GCGGCCGC) synthetisiert. Die PCR-Bedingungen lauten bei Verwendung von 1 ng des Plasmides pGreiner, einer Primerkonzentration von 0.2 µM, 1.5 u Taq-Polymerase (Pharmacia) und 25 µl Reaktionsvolumen in einen Multicycler PTC-200 (MJ RESEARCH, WATERTOWN, USA Research) wie folgt:

| | | |
|---|---|---|
| 1 x | Schritt 1: 4 min | 95°C |
| 30 x | Schritt 2: 30 sec | 95°C |
| | Schritt 3: 30 sec | 57°C |
| | Schritt 4: 2 min | 72°C |
| 1 x | Schritt 5: 5 min | 72°C |

Nach Behandlung des Fragmentes mit den Enzymen *Spe*I und *Not*I wurde das Fragment in den mit *Spe*I und *Not*I linearisierten Vektor pAnja kloniert. Der resultierende Vektor trägt die Bezeichnung pAnja-sek (Fig. 9).

Die Lage der Schnittstelle von *Nae*I (GCC/GGC) wurde so gewählt, daß das GCC-Triplett identisch mit dem Triplett der letzten Aminosäure der Signalsequenz ist. Durch Klonierung des zu exprimierenden Gens in die *Nae*I Schnittstelle der Signalsequenz kann jedes beliebige Protein mit der Signalsequenz in einer funktionellen Weise fusioniert werden. Nach Umklonierung der entsprechenden Kassette bestehend aus Promotor, Signalsequenz und zu exprimierendes Gen in einen binären Vektor kann das Konstrukt in Pflanzen übertragen werden.

### Lokale, elizitorinduzierte Expression des Promotors in Raps

Die für die Produktion transgener Pflanzen vorgesehenen Konstrukte werden zunächst durch ein direktes DNA-Transformationsverfahren (An, 1987) in den *Agrobacterium. tumefaciens* Stamm C58 ATHV überführt. Die Selektion rekombinater *A. tumefaciens* Klone erfolgt unter Verwendung des Antibiotikums Kanamycin (50mg/l). Nachfolgend ist die Transformation beispielhaft für den Vektor pAG5947-trans beschrieben.
Die Reportergenkassette bestehend aus der translationellen Fusion zwischen dem Promotor und dem *gus*-Gen und dem nos-Terminator wird mit Hilfe von *A. tumefaciens* nach Horsch et al. (1985) in den Sommerraps Genotyp Drakkar transformiert. Transgene Pflanzen werden unter Verwendung des Antibiotikums Kanamycin selektioniert. Die Anwesenheit des Promoters in den transgenen Pflanzen kann durch PCR überprüft werden. Die Verwendung der Primer AGATTTTCTTCGTATAGCAGCCAC und GTACCATGATATGCATCATTCTCTT führt zu der Amplifikation eines 269 bp großen DNA-Fragments aus dem Promotor entsprechend der Nukleotidposition 193-461 von Abb.1. Die PCR wird unter Verwendung von 10 ng genomischer DNA, einer Primer-konzentration von 0,2 µM bei einer Annealingtemperatur von 57°C in einen Multicycler PTC-200 (MJ Research, Watertown, USA) durchgeführt. Unter Anwendung der beschriebenen Techniken wurden mit dem binären Vektor pAG5947-trans drei unabhängige Rapstransformanten, die die Bezeichnung AG5947-t38, AG5947-t48 und AG5947-t49 tragen, gewonnen.

### Expressionsverhalten des Promotors in transgienen Pflanzen

### Elizitorinduktion des Promotors in Blättern, Sproß und Wurzeln transgener Rapspflanzen

Blätter werden von den Transformanten AG5947-t48 und AG5947-t49 sowie der nicht-transgenen Ausgangslinie Drakkar abgenommen und für die Elizitorinduktion in 120mm großen Petrischalen in 50 ml Induktionsmedium (LS-Medium ohne Sucrose, 5 mM MES pH 6.1, 1 µ/ml *Rhizopus Pektinase* EC 3.2.1.15 von Sigma Chemical CO.) oder in Kontrollmedium (LS-Medium ohne Sucrose (Linsmaier and Skoog, 1965), 5 mM MES pH 6.1) 16h bei 24°C inkubiert. Die Rhizopus Pektinase setzt aus den pflanzlichen Zellwänden pektolytische Abbauprodukte frei, die ihrerseits eine elizitierende Wirkung haben. Die Aktivität des Promotors wird durch eine quantitative Bestimmung der β-Glucuronidase-(GUS- Aktivität unter Verwendung des Substrates 4-Methyl-umbelliferyl-glucuronid (MUG) nach Jefferson (1987) bestimmt.

Die nichttransgene Ausgangslinie Drakkar zeigt wie in Tabelle 2 dargestellt im nichtelizitierten und im elizitierten Zustand eine spezifische Glucuronidaseaktivität von 6,68 bzw. 6,61 pMol Mu x min⁻1 x mg⁻1. Die Elizitierung führt in der nichttransgenen Linie zu keiner signifikanten Veränderung der Glucuronidaseaktivität.

Die transgenen Linien AG5947-t48 und AG5947-t49 zeigen im nichtelizitierten Zustand eine spezifische Enzymaktivität von 14,0 bzw. 138,7 pMol Mu x min-1 x mg-1 und nach Elizitierung eine Glucuronidaseaktivität von 247,6 bzw. 1507,9 pMol Mu x min-1 x mg-1. Die Glucuronidaseaktivität der transgenen Linien ist bereits im nichtelizitierten Zustand deutlich höher als bei der nichttransgenen Linie. Unter Einwirkung der Elizitierung kommt es zu einer 17,6 bzw. 16,9-fachen Erhöhung der Glucuronidaseaktivität und damit zu einer Induktion des Reportergens. Der Zuckerrüben-Promoter zeigt somit in Blättern transgener Rapspflanzen eine Elizitor- und damit Pathogeninduzierbarkeit.

Um zu prüfen, ob die Elizitorinduzierbarkeit des Promotors auch in anderen Pflanzenorganen als den Blättern gegeben ist, wurden Sproßsegmente und Wurzeln in die Untersuchungen miteinbezogen. Dazu wurden F2-Pflanzen der Transformante AG5947-t49 unter Gewächshausbedingungen angezogen. Abgetrennte Blätter und Sprosse, die in 5 cm lange Abschnitte geschnitten wurden und gereinigte Wurzeln wurden, wie bereits für Blätter beschrieben, 16 h in pectinasehaltigem Induktionsmedium oder in Kontrollmedium inkubiert. Um den Einfluß der Verwundung während der Probennahme auf das Meßergebnis bewerten zu können, wurden ganze Blätter, Sproßabschnitte und gereinigte Wurzeln auch sofort nach der Probennahme in flüssigem Stickstoff eingefroren. Wie in Tabelle 4 dargestellt, wird durch das Abtrennen der Blätter von der Pflanze, die Reportergenaktivität in den Blättern im Vergleich zu dem Kontrollansatz um das 5,3-fache und durch die Elizitierung zusätzlich um das 72-fache erhöht. Das Abtrennen und Zerschneiden der Sproßachse führt zu einer Erhöhung der GUS-Aktivität um das 8,3-fache und durch die zusätzliche Elizitierung um das 3,5-fache.
Im Fall der Wurzel wurde durch das Abtrennen der Wurzel ein 7,2-facher und durch die Elizitierung eine zusätzliche 9-fache Elizitorinduktion der Reportergenaktivität beobachtet. Der Promotor wird in transgenen Pflanzen in Blättern, dem Sproß und den Wurzeln durch Verwundung und zusätzlich durch Elizitierung aktiviert.

### Induktion des Promotors in Blättern transgener Rapspflanzen durch Salicylsäure- und PMG-Elizitor

Um die Wirkung von Salicylsäure und eines gereinigten PMG-Elizitors aus der Zellwand von *Phytophthora sojae* (Valent, 1978) auf die Promotoraktivität zu untersuchen, werden Blattrondelle mit einem Durchmesser von 14 mm mit Hilfe eines Korkbohrers aus den Blättern von Gewächshauspflanzen ausgestanzt. Die Verwendung von Blattrondellen erleichtert die Aufnahme der Salicylsäure bzw. des Elizitors über den Wundrand in das Blattgewebe. Jeweils 10 Blattrondelle aus Blättern der Transformanten AG5947-t38 und AG5957-t48 werden in 90 mm großen Petrischalen in salicylsäurehaltigem Medium (LS-Medium ohne Sucrose, 5 mM MES pH 7.0, 0.5 mM SA), in PMG-elzitorhaltigem Medium (LS-Medium ohne Sucrose, 5 mM MES pH 7.0, 25 µg Elizitor/ml) oder in Kontrollmedium (LS-Medium ohne Sucrose, 5 mM MES pH 7.0) 16h bei 24°C inkubiert. Die Aktivität des Promotors wird wie bei Jefferson (1987) beschrieben durch eine quantitative Bestimmung der GUS-Aktivität gemessen.

Wie in Tabelle 4 dargestellt, kann für die transgenen Linien AG5947-t38 und AG5957-t48 im Kontrollmedium eine Enzymaktivität von 24,47 bzw. 1,86 pMol Mu x min⁻¹ x mg⁻¹ nachgewiesen werden. Die Inkubation der Blattrondelle in Gegenwart von 0,5 mM SA bzw. von 25 µg/ml PMG-Elizitor führt für die Transformante AG5947-t38 zu einer Reporterenzymaktivität von 485,3 bzw. 187,0 pMol Mu x min⁻¹ x mg ⁻¹ und für die Transformante AG5957-t48 zu einer Enzymaktivität von 17,2 bzw. 9,3 pMol Mu x min⁻¹ x mg⁻¹. Damit wird die spezifische Glucuronidaseaktivität der Transformante AG5947-t38 im Vergleich zu dem Kontrollansatz durch die Salicylsäurebehandlung um das 19,8 -fache und durch den PMG-Elizitor um das 7,7-fache gesteigert. Für die Transformante AG5947-t38 beträgt der Induktionsfaktor für Salicylsäure 9,2 und für PMG-Elizitor 5.

### Histochemischer Nachweis der lokalen Promoteraktivierung

Zur Beschreibung des räumlichen Expressionsverhaltens des Promotors werden Blätter der Rapstransformante AG5947-t49 verwendet. Die Blätter werden von Gewächshauspflanzen abgetrennt und in einer durchsichtigen Plastikkiste auf naßes Filterpapier gelegt. In die Mitte einer jeden Blatthälfte werden 20 µl *Rhizopus* Pektinase (10 u/ml) und Wasser als Kontrolle pipettiert. Die Blätter werden 16 h bei 24°C inkubiert und anschließend wird die GUS-Aktivität histochemisch nachgewiesen. Dazu werden die Blätter mit GUS-Färbelösung (2 mM 5-Bromo-4-Chloro-3-Indoyl-beta-Glucuronid, 50 mM Natriumphosphat pH 7,0, 0,5% Triton X-100, 2 % N, N,-Dimethylformamid) für 15 sec vakuuminfiltriert und anschließend für 5 h bei 37°C inkubiert. Die Blaufärbung des Gewebes zeigt die Bereiche an, in denen hohe GUS-Aktivität vorliegt.
Die elizitierte Blatthälfte der Transformante AG5947-t49 zeigt wie in Fig. 10 dargestellt eine räumlich stark begrenzte Blaufärbung und damit Promotoraktivität um die Auftragsstelle der Pektinase. Die übrigen Gewebebereiche der enzymbehandelten Blatthälfte sowie die wasserinokulierte Blatthälfte der transgenen Linie weisen keine sichtbare GUS-Aktivität auf. Diese Ergebnisse zeigen, daß die Induktion des Promoters auf die Inokulationsstelle mit dem elizitorerzeugenden System beschränkt ist. Der Promotor wird in den Blättern transgener Rapspflanzen lokal durch Elizitoren induziert.

### Infektion transgener Pflanzen

Der Nachweis der Pathogeninduzierbarkeit des Promotors in transgenen Pflanzen erfolgt durch Infektion von Blättern der transgenen Rapslinien AG5947-t48 und AG5947-t49 mit dem Erreger der Umfallkrankheit des Rapses, *Phoma lingam.* Pflanzen der transgenen Linien AG5947-t48 und AG5947-t49 sowie der nichttransgenen Linie Drakkar werden unter Gewächshausbedingungen aus Saatgut angezogen. Nachdem die Pflanzen ein Alter von 6 Wochen erreicht haben, werden pro Pflanze jeweils 2 gleichgroße Blätter mit einem Nagelbrett lokal verwundet, um für den Pilz eine Eintrittspforte zu schaffen. Durch Eintauchen der verletzten Blätter in eine Sporensuspension von *Phoma lingam* (100.000 Sporen/ml) werden die Blätter inokuliert. Für Kontrollzwecke werden Blätter transgener und nichttransgener Pflanzen verwundet und nur in Wasser eingetaucht. Die Pflanzen werden anschließend für 7 Tage unter einem Folientunnel im Gewächshaus bei 25°C und 90-100% Luftfeuchtigkeit inkubiert. Nach 7 Tagen wird der Folientunnel entfernt und nach 10 Tagen wird wie oben beschrieben ein histochemischer Nachweis der Promotoraktivität durchgeführt. Zu diesem Zeitpunkt zeigen die pilzinokulierten Blätter eine 6-8 mm große nekrotische Zone um die Verwundungsstellen während die wasserbehandelten Kontrollen nur eine 1-2 mm große Verbräunungsreaktion um die Wundstelle aufweisen. Die pilzinokulierten Blätter der transgenen Pflanzen zeigen um die Infektionsstellen eine lokale Blaufärbung hervorgerufen durch die örtlich beschränkte Promotoraktivität. Die pilzinfizierten Blätter der nichttransgenen Linie Drakkar sowie die verwundeten und wasserbehandelten Blätter der nichttransgenen und der transgenen Linien hingegen weisen keine Blaufärbung und damit Reportergenaktivität auf.

### Aktivitätszunahme des Promotors in nichtinduzierten und induzierten Blättern transgener Pflanzen mit zunehmenden Pflanzenalter.

Zur Untersuchung der entwicklungsabhängigen Aktivität des Promotors in transgenen Pflanzen wird der Vektor pAG5957-trans in Anlehnung an das für Raps beschriebene Protokoll in *Nicotianum tabacum* cv. SR1 transformiert. Die produzierten Tabaktransformanten tragen die Bezeichnung PR1-52, PR1-54 und PR1-56.
Die Transformante PR1-52 wird vermehrt und in das Gewächshaus überführt. Nachdem die Pflanze eine Grösse von ca. 20 cm erreicht hat, wird die Aktivierbarkeit des Zuckerrübenpromotors in Abhängigkeit von dem Pflanzenalter analysiert. Dazu werden 6 Blätter unterschiedlichen Alters an der Pflanze ausgewählt. Das älteste, tiefstehendste Blatt erhält die Bezeichnung 1 und das jüngste, höchste Blatt die Bezeichnung 6. Aus der Blatthälfte eines jeden Blattes werden mit Hilfe eines 14 mm Korkbohrers jeweils 15 gleichgrosse Blattrondelle ausgestanzt. Jeweils 5 Blattrondelle werden in 90 mm großen Petrischalen in salicylsäurehaltigem Medium (LS-Medium ohne Sucrose, 5 mM MES pH 7.0, 0.5 mM SA), in PMG-elzitorhaltigem Medium (LS-Medium ohne Sucrose, 5 mM MES pH 7.0, 1 unit Elizitor/ml) oder in Kontrollmedium (LS-Medium ohne Sucrose, 5 mM MES pH 7.0) 16h bei 24°C inkubiert. Am nächsten Tag werden aus jeder behandelten Blatthälfte nochmals 5 Rondelle für Kontrollzwecke ausgestanzt. Die Aktivität des Promotors in diesen Rondellen sowie in den über Nacht inkubierten Rondellen wird nach Jefferson (1987) durch eine quantitative Bestimmung der GUS-Aktivität gemessen. Nach 16 Tagen wird der entsprechende Versuch mit den noch intakten Blatthälften der Blätter 1-6 wiederholt. Die Tabakpflanze ist inzwischen geschosst und ca. 50-60 cm gross.
Der Vergleich der Messwerte zeigt (s. Tabelle 4b), dass die Promotoraktivität in den Kontrollblättern für 5 der 6 analysierten Blättern in der Versuchswiederholung nach 16 Tagen (t=16 d) deutlich höher ist als zu dem ersten Untersuchungszeitpunkt (t = 0 d). Die Promotoraktivität nimmt mit zunehmenden Alter in den transgenen Pflanzen zu. Die Verwundung bzw. Elizitierung bzw. Saliycalsäureanwendung führt in der Reportergenpflanze mit höherem Pflanzenalter in allen untersuchten Blättern zu einer höheren Reportergen- und damit Promotoraktivität. Das Pflanzenalter hat jedoch keinen Einfluss auf die relative Induktion des Promotors durch Verwundung, Elizitierung oder Salicylsäuregabe.

### Aktivität von Promotordeletionsfragmenten in transgenen Pflanzen

Um den Einfluss der Promotordeletionen auf die Reportergenaktivität zu untersuchen werden die Vektoren pAG516 und pAG2339 mit Hilfe von *A. tumefaciens* in Tabak transformiert. Die unter Verwendung von pAG516 produzierten Transformanten tragen die Bezeichnung PR4-19, PR4-21 und PR4-22 sowie die mit Hilfe von pAG2339 erzeugten transgenen Pflanzen die Bezeichnung PR8-2, PR8-3 und PR8-12.
Klonplanzen der Transformanten PR4-19, PR4-21, PR4-22, PR8-2, PR8-3 und PR8-12 sowie der Transformanten PR1-52, PR1-54 und PR1-56 werden in das Gewächshaus überführt. Nachdem die Tabakpflanzen eine Größe von 60 cm erreicht haben werden 30-40 Blattrondelle aus einem Blatt ausgestanzt und wie beschrieben (s. Aktivitätszunahme des Promotors in nichtinduzierten und induzierten Blättern transgener Pflanzen mit zunehmenden Pflanzenalter.) auf ihre Reaktion gegenüber Wundreiz, Wundreiz und Pektinase bzw. Wundreiz und Salicylsäure analysiert.
Die drei mit pAG5947-trans transformierten Linien PR1-52, PR1-54 und PR1-56 zeigen eine leichte Zunahme der Reportergenaktivität nach Verwundung und eine deutliche Induktion der Promotoraktivität nach Elizitierung bzw. Salicylsäureapplikation (Tabelle 4c). Die mit dem Konstrukt pAG2339 transformierten Pflanzen (PR8-2, PR8-3, PR8-12) zeigen keine signifikante Veränderung der Reportergenaktivität nach Verwundung, Elizitierung bzw. Salicylsäuregabe. Die mit dem Konstrukt pAG516 transformierten Pflanzen PR8-2, PR8-3 und PR8-12 zeigen lediglich eine geringe SA-Induzierbarkeit vor dem Hintergrund einer im Vergleich zu den PR1-Pflanzen sehr geringen Reportergenaktivität.

### Entwicklungsabhängige Aktivität des Promotors in nichtinfizierten Zuckerrüben

Zur Untersuchung der Aktivität des Promotors in den verschiedenen Organen der Zuckerrübe während der Pflanzenentwicklung wird Zuckerrübensaatgut im Feld ausgelegt. Im Verlauf einer mitteleuropäischen Vegetationszeit werden 4, 6, 10, 12, 16 und 22 Wochen nach der Aussaat jeweils 5 vollständige Zuckerrübenpflanzen geerntet. Die Pflanzen zeigen zu keinem Zeitpunkt Krankheitserscheinungen. Gesamtzell-RNA wird nach Logemann et al. 1987 aus den Organen "sink" und "source" Blatt, Petiole, Seitenwurzel und Pfahlwurzel (Rübenkörper) isoliert. Die Aktivität des Promotors wird durch eine RNA-Blot Analyse bestimmt. Als Hybridisierungssonde wird ein Teil des sich an den Promotor anschließenden codierenden Bereichs verwendet. Dazu wird ein 1800 bp großes genomisches DNA-Fragment durch "inverse polymerase chain reaction (IPCR)" aus dem Pflanzengenom des Genotyps 1K0088 amplifiziert.

Die Klonierung der 1800 bp großen Hybridisierungssonde durch IPCR erfolgt in Anlehnung an die Arbeit von Does et al. (1991). Dazu wird zunächst genomische Pflanzen-DNA aus Blättern des Genotyps 1K0088 entsprechend Saghai-Maroof et al. (1984) isoliert. Die genomische DNA (100 ng) wird mit der Restriktionsendonuklease *Bgl*II geschnitten, mit Phenol/Chloroform extrahiert und ethanolgefällt. Die DNA wird anschließend in Ligationspuffer (Life Technologies GmbH, Karlsruhe) aufgenommen und in einem Gesamtvolumen von 15 µl in Gegenwart von 1 u T4 DNA Ligase (Life Technologies GmbH, Karlsruhe) für 4 h bei 16°C entsprechend den Herstellerangaben religiert. Um einen Teil des sich an den 3'-Bereich des Promotors anschließenden codierenden Bereichs zu klonieren, wird eine PCR durchgeführt. Zu diesem Zweck wird der Oligonukleotidprimer R1 mit der Sequenz GTG GCT GCT ATA CGA AGA AAA TCT und der Primer R2 mit der Sequenz ACA CTA TTA TCT ACG CCT CTG ACT für die PCR eingesetzt. Der Primer R1 bindet von Position 5732-5755 der Nukleotidsequenz von Abb.1 und damit 192 bp entfernt vom Transkriptionsstartpunkt des Promotors. Der Primer R2 bindet von Position 5148-5124 der Nukleotidsequenz von Abb. 1 und damit 254 bp hinter der einzigen *Bgl*II Schnittstelle des Promotors. Die PCR Bedingungen lauten bei Verwendung von 5 µl des Ligationsansatzes, einer Primerkonzentration von 0.2 µM, 1.0 u Advantage KlenTaq-Polymerase-Mix (Clontech Laboratories, Heidelberg) und 25 µl Reaktionsvolumen in einem Multicycler PTC-200 (MJ Research, Watertown, MA., USA) folgendermaßen:

| | | |
|---|---|---|
| 1 x | Schritt 1: 4 min | 95°C |
| 35 x | Schritt 2: 30 sec | 95°C |
| | Schritt 3: 30 sec | 57°C |
| | Schritt 4: 4 min | 72°C |
| 1 x | Schritt 5: 5 min | 72°C |

Die Anwendung dieser PCR-Bedingungen führt mit dem Primerpaar R1/R2 zu einem 1800 bp großen DNA-Fragment. Dieses DNA-Fragment kann mit Standardmethoden (Sambroock et al., 1989) in dem Vektor pGEM-T (Promega Corporation, Madison, WI., USA) subkloniert und das klonierte Fragment als Hybridisierungssonde eingesetzt werden.

Für die Untersuchung der entwicklungsabhängigen Prömotoraktivität durch einen RNA-Blot werden jeweils 10 µg Gesamtzell-RNA pro Organ und Zeitpunkt in einem denaturierenden Formaldehyd-Agarosegel, wie bei Sambrook et al. (1989) beschrieben, aufgetrennt. Die elektrophoretisch aufgetrennte RNA wird durch Kapillar-Blot Technik (Sambrook et al., 1989) auf eine Hybond N Nylonmembran (Amersham Pharmacia Biotech, Freiburg) übertragen. Die radioaktive Markierung von 20 ng des 1800 bp grossen DNA-Fragmentes mit 50 µCi ³²P-dATP (6000 Ci/mMol, Amersham Pharmacia Biotech, Freiburg) erfolgt mit Hilfe des Prime-It II Random Primer Kit (Stratagene GmbH, Heidelberg) entsprechend der Herstellerangaben. Die anschließende Hybridisierung des RNA-Filters mit der markierten Sonde geschieht in 20 ml Hybridisierungspuffer (50% Formamid, 5 x SSC, 5 x Dendardts, 1% SDS, 0,1 mg Heringssperma-DNA, 40 mM Natriumphosphatpuffer pH 6,8) bei 42°C in einem Hybridisierungsofen (Biometra GmbH, Göttingen) nach Sambrook et al. 1989. Nach der Hybridisierung wird die Nylonmembran auf einem Röntgenfilm (Kodak BioMax MS, Kodak AG, Stuttgart) in Gegenwart einer Verstärkerfolie (Kodak BioMax MS Intensifying Screen, Kodak AG, Stuttgart) 6-24 h bei-80°C exponiert. Die Entwicklung des Röntgenfilm geschieht in Röntgenfilm-Entwickler und Röntgenfilm-Fixierer (Tetenal Photowerk GmbH und Co., Norderstedt).

Der RNA-Blot zeigt, daß der Promotor im Feld unter Nichtbefallsbedingungen in 4, 6, 10, 12, 16 und 22 Wochen alten Zuckerrüben in Abhängigkeit vom Entwicklungszustand in den einzelnen Pflanzenorganen unterschiedlich stark aktiv ist (Abb. 11). Der RNA-Blot wurde mit einem Phosphoimager (Bioimaging Analyzer BAS 1000, Fujiy Japan) ausgewertet, um die Transkriptakkumulation zu quantifizieren. Die Daten der Quantifizierung sind in Tabelle 4 wiedergegeben.
Die Akkumulation eines mit der durch IPCR-Sonde nachweisbaren Transkriptes und damit die Promotoraktivität ist in den Blättern von 4 Wochen alten Pflanzen nur schwach ausgeprägt, nimmt mit zunehmendem Alter der Pflanzen zu und erreicht nach 22 Wochen ein Maximum. Dieser altersabhängige Anstieg der Expression gilt sowohl für "sink-" als auch für "source"- Blätter. So beträgt die Transkriptmenge in "source"- Blättern nach 22 Wochen das 28-fache im Vergleich zur Transkriptmenge nach 4 Wochen. Für die "sink"-Blätter läßt sich nach 22 Wochen eine 14-fach höhere Transkriptmenge als nach 4 Wochen feststellen. Die Transkriptmengen in den 16 bzw. 22 Wochen alten Blättern liegen deutlich über den Mengen, die zu irgendeinem Zeitpunkt in den anderen untersuchten Organen gefunden werden können. Im Gegensatz zu den Blättern unterliegt die Aktivität des Promotors in den Wurzeln, Seitenwurzeln und Petiolen über die gesamte Vegetationszeit (4-22 Woche) keinen großen Schwankungen. In der Pfahlwurzel ist die Transkriptmenge zum Zeitpunkt 4. Woche so gering wie in den Blättern, steigt bis zur 6. Woche auf das Doppelte an und fällt danach bis zur 16 Woche kontinuierlich ab, um bis zur 22. Woche wieder auf 2,7-fache des 4. Wochen-Wertes anzusteigen. Die Transkriptmenge in den Seitenwurzeln steigt von der 6. bis zur 10. Wochen leicht an und fällt dann bis zur 22. Woche unter den 4. Wochen-Wert ab. In der Petiole bleibt die Transkriptmenge zwischen der 4-16. Woche konstant, um dann bis zur 22. Woche deutlich abzufallen. Die Promotoraktivität ist in den Petiolen bis zur 12. Woche im Vergleich zu den anderen Organen am höchsten. Während die Transkriptmenge in den "sink"-Blättern jedoch kontinierlich und in den "source"-Blättern ab der 12. Woche mit dem Pflanzenalter stark ansteigt, ist dieser Alterseffekt für die Petiolen nicht zu beoabachten.

### Expressionsverhalten unter Befallsbedingungen in Zuckerrüben

### Aktivierung des Promotors in Zuckerrübenblättern korreliert mit dem Befall durch den Blattfleckenerreger Cercospora beticola

Für die Infektion von Zuckerrüben mit dem Blattfleckenerreger C. beticola werden Zuckerrüben des toleranten Genotyps 1K0088 und des anfälligen Genotyps 3S0057 unter Gewächshausbedingungen angezogen. Zwei Wochen vor der geplanten Inokulation werden 20 V8-Gemüsesaftplatten (40% Albani-Gemüsesaft) mit vier verschiedenen C. *beticola* Isolaten beimpft und bei 25°C inkubiert. Unmittelbar vor der Inokulation wird der pilzbewachsene Agar zusammen mit 0,5 I Wasser in einem Hochleistungsrührwerk (UM5 Universal, Stephan) homogenisiert. Die Konzentration an Myzelfragmenten und Pilzsporen in dem Homogenat wird mit Hilfe einer Zählkammer bestimmt. Die Inokulumdichte wird durch Verdünnen mit Wasser auf eine Konzentration von 100.000 Fragmente/ml eingestellt. Das verdünnte Homogenat wird mit Hilfe einer Rückenspritze (Gloria 176T) auf die 12 Wochen alten Zuckerrüben gesprüht. Zu Kontrollzwecken werden Pflanzen mit einem pilzfreiem Agarhomogenat besprüht. Die Pflanzen werden nach der Inokulation für 4 Tage bei 25°C und 95% Luftfeuchtigkeit in einem Gewächshaus inkubiert. Nach dem vierten Tag wird die Luftfeuchtigkeit auf 60-70 % reduziert. Vier, sieben und neun Tage nach der Inokulation werden Blätter von den pilz- und agarinokulierten Pflanzen abgenommen und in flüssigem Stickstoff tiefgefroren. Danach wird Gesamtzell-RNA nach Logeman et al. 1987 isoliert. Die Aktivität des Promotors in den Blättern wird durch eine RNA-Blot Analyse bestimmt. Als Hybridisierungssonde wird das durch IPCR klonierte 1800 bp große genomische DNA-Fragment eingesetzt, das einen Teil des sich an den Promotor anschließenden kodierenden Bereich des Gens enthält. Die Aktivierung des Promotors ist, wie in Fig. 12 dargestellt, in Blättern bereits 4 Tage nach der Inokulation in noch symptomfreien Blättern (-) nachzuweisen. Mit dem Auftreten der Schadsymptome (nach 7 bzw. 9 Tagen) (+ bzw. ++) kommt es zu einer drastischen Akkumulation eines spezifischen Transkriptes. Hierbei ist in stärker befallenen Blättern eine höhere Transkript-Akkumulation zu beobachten als in schwächer befallenen Blättern. Die Korrelation zwischen Genexpression und Befallsgrad gilt sowohl für den *C*. *beticola* toleranten (1K0088) als auch für *C*. *beticola* anfälligen Genotyp (3S0057). Toleranter und anfälliger Genotyp unterscheiden sich jedoch in der Expressionshöhe des Gens in gesunden Blättern. Während eine schwache konstitutive Expression des Gens in gesunden Blättern des toleranten Genotyps zu beobachten ist, ist eine Genexpression in den gesunden Blättern des anfälligen Genotyps nicht nachweisbar.

### Schnelle Aktivierung des Promotors in kleinen und großen Rübenblättern in der frühen Infektionsphase nach C. beticola Befall.

Um die Aktivierung des Promotors in Zuckerrübenblättern während der frühen Infektionsphase zu analysieren wurden Zuckerrüben des Genotyps 1K0088 in einem Versuchsfeld aus Saatgut angezogen. Jeweils eine Versuchsparzelle mit 96 Pflanzen, die 12 Wochen alt sind, wurde mit einem *C*. *beticola* Agar Gemisch bzw. zu Kontrollzwecken nur mit einem Agargemisch inokuliert. Unmittelbar nach der Inokulation und anschließend im täglichen Abstand wurden kleine Blätter (Blattlänge <10 cm) und große Blätter (Blattlänge >20 cm) von den pilzinokulierten und den nur agarinokulierten Pflanzen abgenommen, sofort in flüssigem Stickstoff schockgefroren anschließend bei -80°C eingelagert. Gesamtzell-RNA wurde wie beschrieben isoliert und eine RNA-Blot Analyse durchgeführt. Die entsprechenden RNA-Blots wurden mit einem Phosphoimager (Bio-imaging Analyzer BAS 1000, Fujiy Japan) ausgewertet, um die Transkriptakkumulation zu quantifizieren. Pro Zeitwert und für jeden Blattyp wurde aus den Transkriptmengen, die für Kontroll- und für die infizierten Pflanzen ermittelt wurden, ein Induktionsfaktor berechnet (Tabelle 6). Dieser Induktionsfaktor ist ein Maß für die Aktivierung des Promotors durch Pilzbefall. Die Aktivität des Promotors wurde sowohl in kleinen als auch in großen Blättern schnell induziert. Bereits nach dem 1. Tag, dem ersten Meßzeitpunkt nach der Inokulation, wurde eine 3,8-fache Induktion des Promotors in großen Blättern beobachtet. In kleinen Blättern war eine 5,2-fache Induktion erst am 2. Tag zu beobachten. Eine lichtmikroskopische Untersuchung der Blattoberfläche zeigte, daß die Pilzhyphen erst am 4. Tag nach der Inokulation auf den Blättern auswachsen. Diese Beobachtung legt den Schluß nahe, daß bereits am 1. bzw. 2 Tag Signalsubstanzen des Pilzes erkannt und zur frühen Aktivierung des Promotors führen.

Nach der ersten Aktivierung des Promotors läßt sich in beiden Blattypen wenn auch zeitversetzt ein ähnliches transientes Aktivitätsverhalten beobachten. Die Promotoraktivität fällt am 2. Tag in den großen Blättern und am 3. Tag in den kleinen Blättern stark ab, um dann in den großen Blättern am 3. Tag und in den kleinen Blättern am 4. Tag wieder auf 3,5- bzw. 5,3-fache der Kontrollpflanzen anzusteigen. Nach diesem erneuten Anstieg fällt die Aktivität ein zweites Mal in den großen Blättern am 4.Tag und in den kleinen Blättern am 5. Tag ab. Danach steigt die Promotoraktivität bis zum 6.Tag in den kleinen Blättern wieder auf das 2,2-fache und in den großen Blättern auf das 3,4-fache an. Am 7. Tag ist die Promotoraktivität sowohl für die kleinen als auch für die großen Blättern in den infizierten Pflanzen nicht viel höher als in den nichtinfizierten Pflanzen. Mit dem Auftreten erster sichtbarer Schadsymptome am 8.Tag kommt es dann in den ausschließlich untersuchten großen Blättern zu einer steten Zunahme der Promotoraktivität, die mit einer 26-fach höheren Aktivität in den infizierten Pflanzen am 10. Tag das Maximum in dieser Untersuchung darstellt.

### Vergleich der Promotoraktivität verschiedener Zuckerrübengenotvpen nach der Entwicklung einer C. beticola Infektion.

Drei verschiedene Zuckerrübengenotypen wurden unter Feldbedingungen mit C. beticola infiziert bzw. zu Kontrollzwecken nur mit Agar behandelt, um die Aktivität des Promotors während der sichtbaren Etablierung der Battfleckenkrankheit zu untersuchen. Die Genotypen sind neben der Linie 1K0088, der Spenderpflanze für den Promotor, die Linien 4T0057 und 9B3734. Große Blätter (> 20 cm) wurden unmittelbar vor der Inokulation (0. Tag) bzw. 10, 14 , 17 und 21 Tage nach der Inokulation von jedem Genotyp geerntet und bei -80°C eingefroren. Gesamtzell-RNA wurde für jeden Zeitpunkt aus den Blattproben isoliert und eine RNA-Blot Analyse durchgeführt. Die Transkriptmenge wurde mit Hilfe eines Phosphoimagers quantitativ bestimmt. Die relative Transkriptmenge war, wie in Tabelle 7 dargestellt, für den Genotyp 1K0088 sowohl in den Kontroll- als auch in den infizierte Pflanzen zu jedem Zeitpunkt höher als in den Pflanzen der Genotypen 4T0057 und 9B3734.

### Rhizoctonia solani

Für die Infektion von Zuckerrüben mit dem Wurzelpathogen *Rhizoctonia solani* werden Zuckerrüben des Genotyps 1K0088 zunächst in einem Versuchsfeld aus Saatgut angezogen. Die Anzucht im Feld garantiert die Ausbildung einer kräftigen Hauptwurzel, die bei der Anzucht unter Gewächshausbedingungen nicht gegeben ist. Nach 3 Monaten werden die Pflanzen vorsichtig ausgegraben, einzeln in erdgefüllte 10 l Plastikeimer überführt und für weitere 2 Monate im Gewächshaus kultiviert.

Die Pilzinfektion erfolgt an 5 Monate alten Zuckerrüben in einer Phytozelle. Dazu werden jeweils 5 Löcher mit einem Durchmesser von 1 cm in 5 cm Abstand um die Rübe in die Erde gedrückt. Die Löcher werden mit *R. solani* bewachsenen Gerstenmehl gefüllt. Im Fall der Kontrollpflanzen werden die Inokulationslöcher nur mit Gerstenmehl gefüllt. Die Pflanzen werden bei 25°C, guter Bewässerung und einem 16/8 stündigen Licht/Dunkelwechsel inkubiert.

Nach 14, 20 und 45 Tagen werden jeweils 3 pilzinokulierte und 3 Kontrollpflanzen ausgegraben und die Rübenkörper gesäubert. Während die infizierten Pflanzen nach 14 Tagen noch symptomfrei waren, zeigten sie nach 20 bzw. 45 Tagen deutliche unterirdische Krankheitssymptome. Die Peripherie der Rübenkörper war durch eindringende Pilzhyphen im Durchschnitt 1 bzw. 3 cm tief verbräunt. Die Kontrollpflanzen hingegen zeigten zu keinem der untersuchten Zeitpunkte Krankheitssymptome. Aus jedem Rübenkörper wurde eine 1 cm dicke Gewebescheibe herausgeschnitten. Die Rübenscheiben der infizierten und nichtinfizierten Pflanzen wurden jeweils zu einer Mischprobe zusammengefaßt und Gesamtzell-RNA wurde nach Logemann et al. (1987) isoliert. Jeweils 10 µg Gesamtzell-RNA wurden durch einen RNA Blot Analyse untersucht. Die anschließende Hybridisierung zeigte, daß in den Kontrollpflanzen nach 14, 20 und 45 Tagen keine Transkripte nachzuweisen waren (Fig. 13). Im Fall der infizierten Pflanzen konnten erste Transkripte nach 20 Tagen nachgewiesen werden. Die Transkriptbildung nach 45 Tagen war noch deutlicher ausgebildet. Zum Zeitpunkt 14 Tage waren keine Transkripte nachweisbar. Der Zeitpunkt und die Intensität der Transkriptbildung korreliert mit dem Ausmaß der sichtbaren Krankheitssymptome, d. h. der Promotor wird durch R. *solani*-Befall spezifisch in der Rübenwurzeln induziert. Ein stärkerer Pilzbefall führt zu einer stärkeren Aktivierung des Promotors in der Pflanze.

### Aktivierung des Promotors in Blättern von Zuckerrüben durch Verwundung und Salicylsäure

Um die Reaktion des Promotors in Zuckerrübenblättern auf abiotische Reize wie Verwundung bzw. auf die Wirkung von Resistenzinduktoren wie Salicylsäure zu untersuchen, wurden Blattrondelle (1 cm Durchmesser) aus 12 Wochen alten Zuckerrüben mit Hilfe eines Korkbohrers ausgestanzt. Jeweils 100 Blattrondelle wurden in Kontrollmedium (LS-Medium ohne Sucrose, 5 mM MES pH 7.0) oder in salicylsäurehaltigem Medium (LS-Medium ohne Sucrose, 5 mM MES pH 7.0, 2.0 mM SA) 16 h bei 24°C inkubiert. Gesamtzell-RNA wurde isoliert und eine RNA-Blot Analyse wie zuvor beschrieben durchgeführt. Die Intensität der Hybridisierungssignale auf dem RNA-Blot wurde mit Hilfe eines Phosphoimager quantifiziert. Die Promotoraktivität der Rondelle in dem Kontrollansatz ist über die Versuchszeit nicht konstant (Tabelle 8). Bezogen auf den 0 h-Wert fällt die Promotoraktivität im Kontrollansatz nach 3 h leicht ab, um dann über 6 h und 11 h bis zum 24 h Wert anzusteigen. Nach 24 h ist die Transkriptmenge im Kontrollansatz ca. 3 fach höher als zu Versuchsbeginn (0 h). Dieser Anstieg der Promotoraktivität ist auf die mit dem Ausstanzen der Blattrondelle verbundene Verrundung des Blattgewebes zurückzuführen. Der Promotor zeigt somit eine Wundinduzierbarkeit in den Blättern.
In dem salicylsäurehaltigen Medium kommt es ebenfalls zu einem Anstieg der Transkriptmenge in Abhängigkeit von der Zeit (Tabelle 8). Allerdings ist die Transkriptmenge abgesehen von dem Zeitpunkt 0 h zu allen anderen Versuchszeitpunkten deutlich höher als in den Kontrollansätzen. So beträgt die Transkriptmenge nach 24 h ca. das 8,5-fache der 0 h-Wertes. Ursache für die höhere Aktivität des Promotors in dem salicylsäurehaltigem Medium ist eine Induzierbarkeit des Promotors durch Salicylsäure.

**Tabelle 1**

| Übersicht über die durch PCR-Techniken erzeugten Promotorderivate | | | |
|---|---|---|---|
| Bezeichnung und Bindungsstelle der Primer in der Promotorsequenz von Abb.1 | | Bezeichnung und Grösse der PCR-Produkte (in Klammern) | Plasmidbezeichnung und Sequenzbereich der Promotorderivate nach Subklonierung des nebenstehenden PCR-Produkts1 |
| P0 | (5941-5947) | | |
| P4480 | (1464-1489) | P0/P4480 (4503 bp) | pA4480, 1468-5947 |
| P4047 | (1894-1921) | P0/P4047 (4073 bp) | pA4047, 1901-5947 |
| P3017 | (2920-2949) | P0/P3017 (3047 bp) | pA3017, 2931-5947 |
| P2661 | (3282-3311) | P0/P2661 (2685 bp) | pA2661, 3286-5947 |
| P2339 | (3600-3629) | P0/P2339 (2366 bp) | pA2339, 3609-5947 |
| P1889 | (4054-4079) | P0/P1889 (1913 bp) | pA1889, 4059-5947 |
| P1777 | (4164-4193) | P0/P1777 (1800 bp) | pA1777, 4171-5947 |
| P1777* | (4164-4193) | P0/P1777* (1800 bp) | pA1777*, 4171-5947 |
| P814 | (5124-5194) | P0/P814 (843 bp) | pA814, 5134-5947 |
| P368 | (5570-5599) | P0/P368 (397 bp) | pA368, 5580-5947 |

| | | | |
|---|---|---|---|
| ¹ PCR-Fragmente wurden mit den Restriktionsendonukleasen SalI und Smal geschnitten und in den mit *Sal*I und *Sma*I behandelten Vektor pBluescriptII KS+ kloniert. | | | |

**Tabelle 2**

| Elizitorinduzierbarkeit des Zuckerrüben-Promotors in transgenen Rapspflanzen | | | |
|---|---|---|---|
| Blätter von transgenen (Transformationsvektor AG5947-t) und nichttransgenen Gewächshauspflanzen werden in Gegenwart (Induktionsmedium) oder Abwesenheit (Kontrollmedium) eines elizitorfreisetzenden Enzyms 16 h inkubiert. Die Aktivität des Promotors wird durch eine quantitative Bestimmung der ß-Glucuronidaseaktivität gemessen. | | | |
| Genotyp | Kontrollmedium Spezifische GUS-Aktivität (pMol Mu x min⁻¹ x mg⁻¹) | Induktionsmedium Spezifische GUS-Aktivität (pMol Mu x min⁻¹ x mg⁻¹) | Induktionsfaktor |
| Nichttransgene | 6,68 | 6,61 | 1,0 |
| Linie Drakkar | | | |
| AG5947-t48 | 14,0 | 247,6 | 17,7 |
| AG5947-t49 | 138,7 | 1507,9 | 16,9 |

**Tabelle 3**

| Aktivierung des Promotors in Blättern, Sproß und Wurzeln der transgenen Rapslinie AG5947-t49 nach Verwundung und Elizitierung durch Pektinase. | | | | | |
|---|---|---|---|---|---|
| | Kontrolle | Abgetrennt | Abgetrennt+ Elizitor | Wund-Induktion² | Elizitor-Induktion³ |
| | Spez. GUS- Aktivität1 | Spez. GUS- Aktivität1 | Spez. GUS- Aktivität 1 | | |
| Blatt | 14,3 | 75,5 | 5404,0 | 5,3 | 72,0 |
| Spross | 158,0 | 1315,4 | 4546,3 | 8,3 | 3,5 |
| Wurzel | 72,0 | 520,0 | 4714 | 7,2 | 9,0 |

| | | | | | |
|---|---|---|---|---|---|
| ¹ Spezifische gus-Aktivität: pMol Mu x min¹ x mg⁻¹ ² Verhältnis der spezifischen GUS-Aktivität der abgetrennten Blätter zu der spez. Aktivität der Kontrollblätter. ³ Verhältnis der spezifischen GUS-Aktivität der abgetrennten Blätter zu der spez. Aktivität der abgetrennten und elizitierten Blätter. | | | | | |

**Tabelle 4**

| Induktion des Zuckerrüben-Promotors in Blättern transgener Rapspflanzen durch Salicylsäure- und PMG-Elizitor | | | | |
|---|---|---|---|---|
| | AG5947-t38 Spezfische GUS-Aktivität¹ | Induktion | AG5947-t48 Spezifische GUS-Aktivität¹ | Induktion |
| Kontrolle | 24,47 | | 1,86 | |
| SA (0,5 mM) | 485,3 | 19,8 | 17,2 | 9,2 |
| PMG-Elizitor (25 µg/ml) | 187,0 | 7,7 | 9,3 | 5,0 |

| | | | | |
|---|---|---|---|---|
| ¹ Spezifische GUS-Aktivität: pMol Mu x min⁻¹ x mg⁻¹ | | | | |

**Tabelle 4b**

| Anstieg der Promotoraktivität in Blättern einer transgenen PR1-52 Tabakpflanze mit zunehmenden Pfanzenalter. | | | | | | | |
|---|---|---|---|---|---|---|---|
| | Kontrolle | Abgetrennt | Abgetrennt + Elizitor | Abgetrennt + SA | Wund-Induktion¹ | Elizitor-Induktion² | SA-Induktion³ |
| | Spez.GUS-Aktivität* | Spez.GUS-Aktivität* | Spez. GUS-Aktivität* | Spez. GUS-Aktivität* | | | |
| Blatt 1 | | | | | | | |
| t = 0 d | 59,46 | 238,00 | 327,9 | 314,92 | 4,0 | 5,5 | 5,3 |
| t = 16 d | 28,25 | 325,17 | 394,28 | 861,82 | | 14,0 | 30,5 |
| Blatt 2 | | | | | | | |
| t = 0 d | 14,98 | 116,37 | 130,17 | 205,53 | 7,8 | 8,7 | 13,6 |
| t = 16 d | 76,75 | 160,12 | 1166,49 | 2358,18 | 2,1 | 15,2 | 30,7 |
| Blatt 3 | | | | | | | |
| t = 0 d | 10,38 | 83,31 | 270,19 | 225,07 | 8,0 | 26,0 | 21,7 |
| t = 16 d | 63,38 | 204,38 | 863,05 | 555,62 | 3,2 | 13,6 | 8,8 |
| Blatt 4 | | | | | | | |
| t = 0 d | 4,59 | 48,87 | 172,53 | 117,97 | 10,6 | 37,6 | 25,7 |
| t = 16 d | 35,06 | 141,6 | 482,88 | 1561,3 | 4,0 | 13,8 | 44,5 |
| Blatt 5 | | | | | | | |
| t = 0 d | 4,78 | 58,24 | 185,6 | 140,37 | 12,2 | 38,8 | 29,4 |
| t = 16 d | 134,96 | 426,23 | 512,63 | 1220,71 | 3,2 | 3,8 | 9,0 |
| Blatt 6 | | | | | | | |
| t = 0 d | 0,33 | 37,11 | 121,41 | 106,35 | 111,5 | 364,6 | 319,4 |
| t = 16 d | 21,33 | 146,74 | 1069,58 | 1003,47 | 6,9 | 50,1 | 47,0 |

| | | | | | | | |
|---|---|---|---|---|---|---|---|
| * Spezifische GUS-Aktivität: pMol Mu x min⁻¹ x mg⁻¹ ¹ Verhältnis der spezifischen GUS-Aktivität der abgetrennten Blätter zu der spez. GUS-Aktivität der frisch geernteten Kontrollblätter. ² Verhältnis der spezifischen GUS-Aktivität der frisch geernteten Kontrollblätter zu der spez. GUS-Aktivität der abgetrennten und elizitierten Blätter. ³ Verhältnis der spezifischen GUS-Aktivität der frisch geernteten Kontrollblätter zu der spez. GUS-Aktivität der abgetrennten und Salicylsäure behandelten Blätter. | | | | | | | |

**Tabelle 4c**

| Promotoraktivität der Deletionskonstrukte pAG 516 und pAG2339 sowie der translationellen Fusion pAG5947-trans in Blättern transgener Tabakpflanzen. | | | | | | | |
|---|---|---|---|---|---|---|---|
| Die Pflanzen PR1-52, PR1-54 und PR1-56 wurden mit dem Konstrukt pAG5947-trans transformiert. Weiterhin wurden die Pflanzen PR4-19, PR4-21 und PR4-22 mit pAG516 und die Pflanzen PR8-2, PR8-3 und PR8-12 mit dem Konstrukt pAG2339 transformiert. SR1 ist die nichttransgene Ausgangslinie. | | | | | | | |
| | Kontrolle | Abgetrennt | Abgetrennt + Elizitor | Abgetrennt + SA | Wund-Induktion¹ | Elizitor-Induktion² | SA-Induktion³ |
| | Spez.GUS-Aktivität* | Spez. GUS-Aktivität* | Spez. GUS-Aktivität* | Spez. GUS-Aktivität* | | | |
| PR1-52 | 15,1 | 35,96 | 199,21 | 218,93 | 2,4 | 13,2 | 14,5 |
| PR1-54 | 20,87 | 43,29 | 216,38 | 167,83 | 2,1 | 10,4 | 8,0 |
| PR1-56 | 80,07 | 475,82 | 938,61 | 661,19 | 5,9 | 11,7 | 8,3 |
| PR4-19 | 7,4 | 2,9 | 4,0 | 16,6 | 0,4 | 0,5 | 2,3 |
| PR4-21 | 36,6 | 38,8 | 23,7 | 21,3 | 1,1 | 0,7 | 0,6 |
| PR4-22 | 5,4 | 2,7 | 4,0 | 19,3 | 0,5 | 0,8 | 3,6 |
| PR8-2 | 8,4 | 0 | 0 | 1,7 | 0 | 0 | 0,2 |
| PR8-3 | 26,9 | 31,0 | 0 | 0 | 1,2 | 0 | 0 |
| PR8-12 | 0 | 5,3 | 0 | 0 | 0 | 0 | 0 |
| SR1 | 7,9 | 0 | 2,8 | 6,2 | 0 | 0,4 | 0,8 |

| | | | | | | | |
|---|---|---|---|---|---|---|---|
| * Spezifische GUS-Aktivität: pMol Mu x min⁻¹ x mg⁻¹ ¹ Verhältnis der spezifischen GUS-Aktivität der abgetrennten Blätter zu der spez. GUS-Aktivität der frisch geernteten Kontrollblätter. ² Verhältnis der spezifischen GUS-Aktivität der frisch geernteten Kontrollblätter zu der spez. GUS-Aktivität der abgetrennten und elizitierten Blätter. ³ Verhältnis der spezifischen GUS-Aktivität der frisch geernteten Kontrollblätter zu der spez. GUS-Aktivität der abgetrennten und Salicylsäure behandelten Blätter. | | | | | | | |

**Tabelle 5:**

| Vergleich der entwicklungsabhängigen Aktivität des Promotors in 5 verschiedenen Organen der Zuckerrübe | | | | | | |
|---|---|---|---|---|---|---|
| Gesamtzell-RNA wurde nach der Aussaat zu verschiedenen Entwicklungszeitpunkten (4, 6, 10 12, 16, 22 Wochen) aus "sink"- und "source"-Blättern, aus Petiolen, Pfahlwurzeln und Seitenwurzeln von Zuckerrüben isoliert und durch eine RNA-Blot Analyse untersucht. Als Hybridisierungssonde wurde ein 1800 bp großes durch IPCR gewonnenes DNA-Fragment eingesetzt. Die durch die Promotoraktivität gebildete Transkriptmenge wurde mit Hilfe eines Phosphoimager quantifiziert und in der Tabelle für jeden Versuchszeitpunkt dargestellt. | | | | | | |
| Organ | 4 Wochen | 6 Wochen | 10 Wochen | 12 Wochen | 16 Wochen | 22 Wochen |
| "source" Blatt | 43¹ | 98 | 109 | 86 | 774 | 1191 |
| "sink" Blatt | 36² | 44 | 74 | 136 | 356 | 502 |
| Petiole | n.b.³ | 162 | 195 | 198 | 201 | 61 |
| Pfahlwurzel | 49 | 100 | 78 | 38 | 42 | 132 |
| Seitenwurzel | n.b.³ | 83 | 140 | 62 | 74 | 46 |

| | | | | | | |
|---|---|---|---|---|---|---|
| ¹ Als "source" Blatt wurde bei den 4 Wochen alten Zuckerrüben das erste Blattpaar gewählt. ² Als "sink" Blatt wurden bei den 4 Wochen alten Zuckerrüben die Keimblätter ausgewählt. ³ n.b. = nicht bestimmt. | | | | | | |

**Tabelle 6**

| Induktion des Zuckerrüben-Promoters in kleinen und grossen Zuckerrübenblättern nach *Cercospora beticola* Befall | | |
|---|---|---|
| Gesamtzell-RNA aus pilzinokulierten und Kontrollpflanzen wurde durch eine RNA-Blot Analyse untersucht. Die durch die Promotoraktivität gebildete Transkriptmenge wurde mit Hilfe eines Phosphoimager quantifiziert. Der Quotient aus der Transkriptmenge der Kontrollpflanzen und der Transkriptmenge der inokulierten Pflanzen wurde als Induktionsfaktor bezeichnet (1 = keine Induktion, 2 = zweifache Transkriptmenge usw. ). | | |
| Zeitpunkt nach Inokulation (Tage) | Kleine Blätter (<10 cm) Induktionsfaktor | Große Blätter (>20 cm) Induktionsfaktor |
| 0 | 1,1 | 1,5 |
| 1 | 1,0 | 3,8 |
| 2 | 5,2 | 1,4 |
| 3 | 1,1 | 3,5 |
| 4 | 5,3 | 1,6 |
| 5 | 1,6 | 2,0 |
| 6 | 2,2 | 3,4 |
| 7 | 1,1 | 1,1 |
| 8 | | 1,9 |
| 9 | | 2,9 |
| 10 | | 26,0 |

**Tabelle 7**

| Vergleich der Aktivität verschiedener Allele des Promotors in drei verschiedenen Zuckerrübengenotypen nach *Cercospora beticola* Befall | | | | | |
|---|---|---|---|---|---|
| Gesamtzell-RNA aus pilzinokulierten und Kontrollpflanzen wurde durch eine RNA-Blot Analyse untersucht. Die durch die Promotoraktivität gebildete Transkriptmenge wurde mit Hilfe eines Phosphoimager quantifiziert und in der Tabelle für jeden Versuchstag dargestellt. Die Ergebnisse der Kontrollpflanzen sind mit (-) und die Ergebnisse der pilzinokulierten Pflanzen mit (+) gekennzeichnet. n.b. = nicht bestimmt. | | | | | |
| Genotyp | 0 Tage | 10 Tage | 14 Tage | 17 Tage | 21 Tage |
| 4T0057 (-) | 323¹ | 332 | 225 | 254 | 633 |
| 4T0057 (+) | n.b. | 756 | 790 | 1920 | 3444 |
| 9B3734 (-) | 265 | 277 | 432 | 576 | 477 |
| 9B3734 (+) | n.b. | 781 | 6303 | 7653 | 10685 |
| 1K0088 (-) | 1753 | n.b. | 787 | 4492 | 2762 |
| 1K0088 (+) | n.b. | n.b. | 10152 | 8537 | 22598 |

| | | | | | |
|---|---|---|---|---|---|
| ¹ Messwerte sind in PSL (photo stimulated luminescence)-Einheiten angegeben. | | | | | |

**Tabelle 8**

| Aktivierung des Promotors in Blättern von Zuckerrüben durch Verwundung und Salicylsäure | | |
|---|---|---|
| Blattrondelle von Zuckerrüben wurden entsprechend der angegebenen Zeitdauer in Flüssigmedium in Gegenwart und in Abwesenheit von 2,0 mM Saliyclsäure inkubiert. RNA wurde aus den Rondellen isoliert und eine RNA-Blot Analyse durchgeführt. Die durch die Promotoraktivität gebildete Transkriptmenge wurde mit Hilfe eines Phosphoimager quantifiziert und in der Tabelle für jeden Versuchszeitpunkt dargestellt. | | |
| Zeit (h) | Kontrolle | Kontrolle + 2,0 mM Salicylsäure |
| 0 | 107¹ | 81 |
| 3 | 61 | 107 |
| 6 | 214 | 284 |
| 11 | 211 | 513 |
| 24 | 339 | 688 |

| | | |
|---|---|---|
| ¹ Messwerte sind in PSL (photo stimulated luminescence)-Einheiten angegeben. | | |

### Literatur:

Altschul, S.F. et al. (1990). Basic Local Alignment search tool, J.Mol. Biol. 215: 403-410
An, G. (1987). Binary Ti vectors für plant transformation and promoter analysis. Methods Enzymol. 153, 292-305.
DE 4207358 A1 (Institut für Genbiologische Forschung Berlin GmbH). Expressionskassette und Plasmide zur schliesszellenspezifischen Expression und ihre Verwendung zur Herstellung transgener Pflanzenzellen und Pflanzen.
da Costa e Silva, O., Klein, L., Schmelzer, E., Trezzini, G. F., and Hahlbrock, K. (1993). BPF-1, a pathogen-induced DNA-binding protein involved in the plant defense response. Plant Journal 4 (1), 125-135.
De Greve, H., Dhaese, P., Seurinck, J., Lemmers, M., Van Montagu, M., and Schell, J. (1982). Nucleotide sequence and transcript map of the Agrobacterium tumefaciens Ti plasmid-encoded Octopine synthase gene. J Mol. Appl. Genet. 1 (6), 499-511.
Depicker, A., Stachel, S., Dhaese, P. Zambryski, P., and Goodman, H. M. (1982). Nopaline synthase: Transcript mapping and DNA sequence. J Mol. Appl. Genet. 1 (6), 561-573.
Does, M. P., Dekker, B. M. M., de Groot, M. J. A., and Offringa, R. (1991). A quick method to estimate the T-DNA copy number in transgenic plants at an early stage after transformation, using inverse PCR. Plant Mol. Biol. 17, 151-153.
Dzelzkälns, V. A., Thorsness, M. K., Dwyer, K. G., Baxter, J. S., Balent, M. A., Nasrallah, M. E., and Nasrallah, J. B. (1993). Distinct cis-acting elements direct pistil-specific and pollenspecific activity of the Brassica S locus glycoprotein gene promoter. Plant Cell 5, 855-863.
EP 0344029 B1. (Plant Genetic Systems, N. V. 1040 Brussel). Plants with modified stamen cells.
Fennell von, et al., Plant Cell Rep. 11 (1992), 567-570
Gottschalk, T. E., and Mikkelsen, J. D. (1998). Immunolocalization and characterization of a beta-1,3-glucanase from sugar beet, deduction of ist primary structure and nucleotide sequence by cDNA and genomic cloning. Plant Science 132, 153-167.
Greiner, S., Krausgrill, S., and Rausch, T. (1998). Cloning of a tobacco invertase inhibitor. Plant Physiol. 116, 733-742.
Höfgen R. & Hesse H.: DE 19607697, erteilt 09-04-1998 / WO 97/32027,veröff. 04-09-199
Horsch, R. B., Fry, J. E., Hoffmann, N. L., Rogers, S. G., Fraley, R. T. (1985). A simple and general method for transferring genes into plants. Science 227, 1229-1231.
Jähne et at., Theor. Appl. Genet. 89 (1994), 525-533)
Jefferson, R. A. (1987). Assaying chimeric genes in plants: The GUS gene fusion system. Plant Mol Biol. Rep: 5, 387-405.
Linsmaier, E. M., and Skoog, F. (1965). Organic growth factor requirements of tobacco tissue cultures. Plant Physiol. 18, 100-127.
Logemann, J., Schell, J., and Willmitzer, L. (1987). Improved method for the isolation of RNA from plant tissue. Anal. Biochem. 163, 16-20.
Lois, R., Dietrich, A., Hahlbrock, K., and Schulz, W. (1989). A phenylalanine ammonia-lyase gene from parsley: Structure, regulation and identification of elicitor and light-responsive cis-acting elements. EMBO J. 8, 1641-1648.
Odell, J. T.Nagy, F., and Chua, N.-H. (1985). Identification of DNA sequences required for activity ofthe cauliflower mosaic virus 35S.promoter. Nature 313, 810-812.
Ohl, S., Hedrick, S. A., Chory, J., and Lamb, C. J. (1990). Functional properties of a phenylalanine ammonia-lyase promoter from Arabidopsis. Plant Cell 2, 837-848.
Ohme-Takagi, M., and Shinshi, H. (1995). Ethylene-inducible DNA-binding proteins that interact with an ethylene-responsive element. Plant Cell 7, 173-182.
Raventós, D., Jensen, A. B., Rask, M.-B., Casacuberta, J. M., Mundy, J., and San Segundo, B. (1995). A 20 bp cis-acting element is both necessary and sufficient to mediate elicitor response of a maize PRms gene. Plant J. 7(1), 147-155.
Rushton, P. J., Torres, J. T., Parniske, M., Wernert, P., Hahlbrock, K., and Somssich, I. (1996). Interaction of elicitor-induced DNA binding proteins with elicitor response elements in the promoters of parsley PR1 genes. EMBO J. 15, 5690-5700.
Rushton, P. J., and Somssich, I. E. (1998). Transcriptional control of plant genes responsive to pathogens. Curr. Opion. In Plant Biol. 1, 311-315.
Saghai-Maroof, M. A., Solimanm, K. M., Jorgensen, R. A., ans Allard, R. W. (1984). Ribosomal DNA spacer length polymorphism in barley: mendelian inheritance, chromosomal location and population dynamics. Proc. Natl. Acad. Sci. USA 81, 8014-8018.
Sambrook , J., Fritsch, E..F., and Maniatis, T (1989). In Molecular Cloning, A Laboratrory Manual. (Cold Spring Harbor Laboratory Press, New York).
Shah, J., and Klessig, D. F. (1996). Identification of a salicylic acid-responsive element in the promoter of the tobacco pathogenesis-related rn-1,3-glucanase gene, PR-2d. Plant J. 10, 1089-1101.
Stoeger et al. Plant Cell Rep. 14 (1995), 273-278
US 005608150A (Monsanto Company). Fruit specific promoters.
Valent, B. (1978). Dissertation, University of Colorado, Seite 8.
Velten, J., Velten, L., Hain, R., and Schell, J. (1984). Isolation of a dual promoter fragment from Ti plasmid of Agrobacterium tumefaciens. EMBO J. 12, 2723-2730.
WO 92/17591 (Danisco A/S) A Plant Chitinase gene and use thereof.

## Patentansprüche

1. Isolierte Nukleotid-Sequenz mit der Eigenschaft eines Promotors zur Erhöhung der Abwehrreaktion einer Pflanze gegen Pathogenbefall, **dadurch gekennzeichnet, daß** die Nukleotid-Sequenz mindestens zwei verschiedene cis-Elemente, ausgewählt aus der Gruppe der cis-Elemente
a) L-Box oder L-Box ähnliche Sequenz, welche die Hexanukleotid-Sequenz CCTAc/aC enthält und in mindestens einem weiteren Nukleotid mit der L-Box übereinstimmt,
b) GCC-Box
c) W-Box mit einer Hexanukleotidsequenz TTGACC
enthält, wobei eine erste W-Box in normaler und eine zweite W-Box in reverser, komplementärer Orientierung vorliegt.

2. Nukleotid-Sequenz nach Anspruch 1, **dadurch gekennzeichnet, daß** einzelne cis-Elemente in Wiederholungen, unabhängig von ihrer Orientierung vorliegen.

3. Nukleotid-Sequenz nach Anspruch 1 oder 2, **dadurch gekennzeichnet, daß** das cis-Element gemäß a) in dreifacher Wiederholung vorliegt.

4. Nukleotid-Sequenz nach Anspruch 2 bis 3, **dadurch gekennzeichnet, daß** das cis-Element gemäß b) zweifach und in reverser, komplementärer Orientierung zur TATA-Box vorliegt.

5. Nukleotid-Sequenz nach Anspruch 1 bis 4, **dadurch gekennzeichnet, daß** die Nukleotid-Sequenz ein weiteres cis-Element enthält, welches für eine Salicylsäure-Induzierbarkeit verantwortlich ist.

6. Nukleotid-Sequenz nach Anspruch 5, **dadurch gekennzeichnet, daß** es sich bei dem weiteren cis-Element um ein Fragment handelt, das ein SAR-Element enthält.

7. Nukleotid-Sequenz nach Anspruch 6, **dadurch gekennzeichnet, daß** sich das SAR-Element in reverser, komplementärer Orientierung in direkter Nähe zu einer W-Box befindet.

8. Nukleotid-Sequenz nach einem der vorhergehenden Ansprüche mit einer Sequenz gemäß Figur 1.

9. Nukleotld-Sequenz nach Anspruch 1 bis 8 zur Sekretion exprimierter Proteine in Apoplasten, **dadurch gekennzeichnet, daß** die Nukleotid-Sequenz transkriptionell mit der Signalsequenz des apoplastischen Invertase-Inhibitors aus Tabak fusioniert wurde.

10. Nukleotid-Sequenz nach Anspruch 1 bis 8, die mit einer Nukleotid-Sequenz am 3'-Bereich translationell erweitert wurde.

11. Nukleotid-Sequenz nach Anspruch 10, **dadurch gekennzeichnet, daß** eine translationelle Fusion mit Hilfe einer Nukleotid-Sequenz aus der Sequenz nach Fig. 5 durchgeführt wurde.

12. Genkonstrukt, das ein Gen enthält, das unter der Kontrolle eines Promotors steht, wobei das Gen ein Pathogenabwehrgen ist und der Promotor eine Nukleotid-Sequenz nach einem der Ansprüche 1 bis 11 aufweist.

13. Verfahren zur Erhöhung der Abwehrreaktion einer Pflanze gegen Pathogenbefall, **dadurch gekennzeichnet, daß** man in dem Genom der Pflanze ein Genkonstrukt nach Anspruch 12 stabil etabliert.

14. Transgene Pflanzenzelle, in die eine Nukleotld-Sequenz nach einem der Ansprüche 1 bis 11 oder ein Genkonstrukt nach Anspruch 12 stabil integriert wurde.

15. Transgene Pflanze, enthaltend eine Pflanzenzelle nach Anspruch 14.

16. Transgene Pflanze nach Anspruch 15, **dadurch gekennzeichnet, daß** die Pflanze eine dikotyle Pflanze ist.

17. Transgene Pflanze nach Anspruch 16, **dadurch gekennzeichnet, daß** sie ausgewählt ist aus der Gruppe, umfassend Fabaceae (Pisum, Vicia, Phaseolus, Glycine), Ranunculaceae, Brassicaceae, Chenopodiaceen (Beta), Solanaceen (Nicotiana, Solanum tuberosum), Lycopersicon, Daucus, Gossypium, Helianthus.

18. Transgene Pflanze nach Anspruch 15, **dadurch gekennzeichnet, daß** die Pflanze eine monokotyle Pflanze ist.

19. Transgene Pflanze nach Anspruch 18, **dadurch gekennzeichnet, daß** sie ausgewählt ist aus der Gruppe der Poaceae, umfassend Zea mays, Triticum, Avena, Secale, Oryza.

20. Samen einer transgenen Pflanze, wobei in die Samen eine Nukleotid-Sequenz nach einem der Ansprüche 1 bis 11 oder ein Genkonstrukt nach Anspruch 12 stabil integriert wurde.

## Claims

1. Isolated nucleotide sequence possessing the characteristic of a promoter for elevating the defensive reaction of a plant against pathogen infection, **characterized in that** the nucleotide sequence contains at least two different cis-elements selected from a group consisting of
a) L-Box or L-Box-like sequence, which comprises the hexanucleotide seuqnce CCTAc/aC and corresponds in at least one further nucleotide with the L-Box,
b) GCC-Box,
c) W-Box with the hexanucleotide sequence TTGACC,
wherein a first W-Box is in normal and a second W-Box in reverse complementary orientation.

2. Nucleotide sequence according to claim 1, **characterized in that** individual cis-elements are present in repetitions, in either orientation.

3. Nucleotide sequence according to claim 1 or 2, **characterized in that** the cis-element according to a) lies in three repetitions.

4. Nucleotide sequence according to any of claims 2 to 3, **characterized in that** the cis-element according to b) is twice repeated in reverse complementary orientation to TATA-Box.

5. Nucleotide sequence according to any of claims 1 to 4, **characterized in that** the nucleotide sequence includes a further cis-element which is responsible for the salicylic acid inducibility.

6. Nucleotide sequence according to claim 5, **characterized in that** the further cis-element is a fragment which is a SAR-element.

7. Nucleotide sequence according to claim 6, **characterized in that** the SAR-element is in reverse complementary orientation in close proximity to a W-Box.

8. Nucleotide sequence according to any of preceding claim having the sequence of SEQ ID NO: 1.

9. Nucleotide sequence according to any of claims 1 to 8 for secretion of expressed proteins into apoplasts, **characterized in that** the nucleotide sequence is fused transcriptionally with the signal sequence of the apoplastic invertase-inhibitor from tobacco.

10. Nucleotide sequence according to any of claims 1 to 8, which has been extended translationally with a nucleotide sequence at the 3'-area.

11. Nucleotide sequence according to claim 10, **characterized in that** a translational fusion is carried out with the aid of the nucleotide sequence from the sequence according to FIG. 5.

12. Gene construct, which comprises a gene operably linked to a promoter, wherein the gene is a pathogen defense gene and the promoter comprises the nucleotide sequence according to any of claims 1 to 11.

13. Method for enhancing the defensive reaction of a plant against infections by pathogens, **characterized in that** the method comprises stably establishing a gene construct according to claim 12 in the genome of the plant.

14. Transgenic plant cell, in which the nucleotide sequence according to any of claims 1 to 11 or a gene construct according to claim 12 has been integrated stably.

15. Transgenic plant comprising a plant cell according to claim 14.

16. Transgenic plant according to claim 15, **characterized in that** the plant is a dicotyledonous plant.

17. Transgenic plant according to claim 16, **characterized in that** the plant is selected from the group consisting of Fabaceae (Pisum, Vicia, Phaseolus, Glycine), Ranunculaceae, Brassicaceae, Chenopodiaceen (Beta), Solanaceen (Nicotiana, Solanum tuberosum), Lycopersicon, Daucus, Gossypium, Helianthus.

18. Transgenic plant according to claim 15, **characterized in that** the plant is a monocotyledonous plant.

19. Transgenic plant according to claim 18, **characterized in that** the plant is selected from the group consisting of Poaceae comprising Zea mays, Triticum, Avena, Secale, Oryza.

20. Seeds of the transgenic plant, wherein in the seeds Samen a nucleotide sequence according to any of claims 1 to 11 or a gene construct according to claim 12 has been integrated stably.

## Revendications

1. Séquence nucléotide isolée présentant la propriété d'un promoteur destiné à augmenter la réaction immunitaire d'une plante contre une infestation d'agents pathogènes, **caractérisée en ce que** la séquence nucléotide contient au moins deux différents éléments cis, choisis dans le groupe des éléments cis :
a) séquence L-box ou analogue à L-box, laquelle contient la séquence hexanucléotide CCTAc/aC et qui concorde par au moins un nucléotide supplémentaire avec la L-box,
b) GCC-Box
c) W-Box avec une séquence hexanucléotide TTGACC
une première W-box se présentant dans une orientation normale et une deuxième W-Box se présentant dans une orientation inverse, complémentaire.

2. Séquence nucléotide selon la revendication 1, **caractérisée en ce que** des éléments cis individuels se présentent en répétitions, indépendamment de leur orientation.

3. Séquence nucléotide selon la revendication 1 ou 2, **caractérisée en ce que** l'élément cis selon a) se présente en triple répétition.

4. Séquence nucléotide selon la revendication 2 à 3, **caractérisée en ce que** l'élément cis selon b) se présente en orientation inverse, complémentaire à la TATA-box.

5. Séquence nucléotide selon la revendication 1 à 4, **caractérisée en ce que** la séquence nucléotide contient un élément cis supplémentaire, lequel est responsable d'une inductibilité à l'acide salicylique.

6. Séquence nucléotide selon la revendication 5, **caractérisée en ce que** l'élément cis supplémentaire est un fragment qui contient un élément SAR.

7. Séquence nucléotide selon la revendication 6, **caractérisée en ce que** l'élément SAR se trouve en orientation inverse, complémentaire à proximité directe d'une W-box.

8. Séquence nucléotide selon l'une quelconque des revendications précédentes, avec une séquence selon SEQ ID NO: 1.

9. Séquence nucléotide selon les revendications 1 à 8 pour la sécrétion de protéines exprimées dans des apoplastes, **caractérisée en ce qu'**on a procédé à une fusion transcriptionnelle de la séquence nucléotide avec la séquence de signaux de l'inhibiteur d'invertase apoplastique à partir de tabac.

10. Séquence nucléotide selon les revendications 1 à 8, qui a été élargie par translation en zone 3' avec une séquence nucléotide.

11. Séquence nucléotide selon la revendication 10, **caractérisée en ce qu'**on a procédé à une fusion translationnelle à l'aide d'une séquence nucléotide de la séquence selon la figure 5.

12. Construction génique qui contient un gène se trouvant sous le contrôle d'un géniteur, le gène étant un gène immunitaire contre les agents pathogènes et le promoteur comportant une séquence nucléotide selon l'une quelconque des revendications 1 à 11.

13. Procédé destiné à augmenter la réaction immunitaire d'une plante contre une infestation d'agents pathogènes, **caractérisé en ce que** dans le génome de la plante, on établit de manière stable une construction génique selon la revendication 12.

14. Cellule végétale transgénique dans laquelle on a intégré de manière stable une séquence nucléotide selon l'une quelconque des revendications 1 à 11 ou une construction génique selon la revendication 12.

15. Plante transgénique contenant une cellule végétale selon la revendication 14.

16. Plante transgénique selon la revendication 15, **caractérisée en ce que** la plante est une plante dicotylédone.

17. Plante transgénique selon la revendication 16, **caractérisée en ce qu'**elle est choisie dans le groupe comprenant la famille des Fabaceae (Pisum, Vicia, Phaseolus, Glycine), des Ranunculaceae, des Brassicaceae, des Chenopodiacées (Beta), des Solanacées (Nicotiana, Solanum tuberosum), des Lycopersicons, Daucus, Gossypium, Helianthus.

18. Plante transgénique selon la revendication 15, **caractérisée en ce que** la plante est une plante monocotylédone.

19. Plante transgénique selon la revendication 18, **caractérisée en ce qu'**elle est choisie dans le groupe de la famille des Poaceae, comprenant le Zea mays, le Triticum, l'Avena, le Secale, l'Oryza.

20. Semence d'une plante transgénique, une séquence nucléotide selon l'une quelconque des revendications 1 à 11 ou une construction génique selon la revendication 12 ayant été intégrée de manière stable dans les semences.
